# EUROPEAN PATENT SPECIFICATION

(11) **EP 1 424 051 B1**
(45) Date of publication and mention of the grant of the patent: **31.08.2011**
(21) Application number: 02771742.0
(22) Date of filing: 21.05.2002
(51) Int. Cl.: A61F 13/15, A61F 13/472, A61F 13/551

(54) **INTERLABIAL PAD**
INTERLABIAL-POLSTER
TAMPON INTERLABIAL

(30) Priority: 22.05.2001 JP 2001152403; 27.06.2001 JP 2001195365
(43) Date of publication of application: 02.06.2004
(73) Proprietor: UNI-CHARM CORPORATION, Shikokuchuo-shi Ehime 799-0111 (JP)
(72) Inventor: MIZUTANI, Satoshi, Kanonji-shi Kagawa 769-1602 (JP); YAMAKI, Koichi,, Kanonji-shi, Kagawa 769-1602 (JP); NODA, Yuki, Kanonji-shi, Kagawa 769-1602 (JP)
(74) Representative: Main, Malcolm Charles
(86) International application number: PCT/JP2002/004881
(87) International publication number: WO 2002/094145

(56) References cited:
- EP-A- 0 419 434
- WO-A-95/17148
- WO-A-99/55270
- WO-A1-98/08475
- WO-A1-98/57610
- WO-A1-99/01093
- WO-A1-99/01096
- WO-A1-99/26575
- US-A- 2 331 355
- US-A- 4 595 392

## Description

### Background of the Invention

### Technical Field

The present invention relates to an interlabial pad to be tightly fixed to the interlabial space.

### Background Art

Conventionally, a sanitary napkin and a tampon are used generally as a female sanitary product. Here, there have been great efforts to prevent the leak of menstrual blood from a gap caused by poor adhesion near the ostium vaginae for the sanitary napkin. Moreover, for the tampon, there have been great efforts to relieve the foreign feeling, the discomfort on wearing the sanitary product and the intervaginal wearing trouble due to the nature of the product.

Under such situations, a sanitary product of an interlabial pad has attracted people as a sanitary product positioned between the sanitary napkin and the tampon in recent years. For example, US4595392 discloses an interlabial pad.

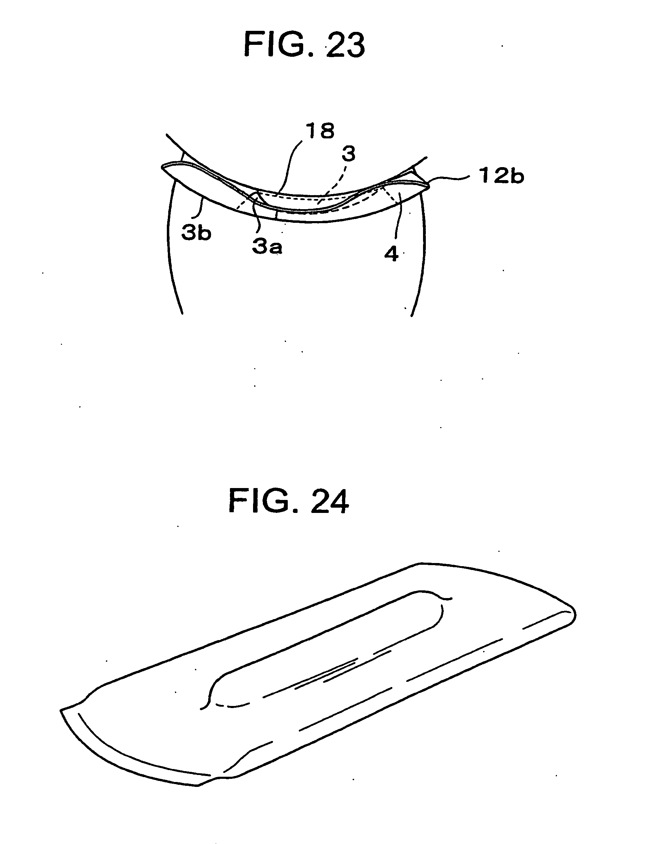

On the other hand, Japanese Utility Model Publication No. Hei 5 - 18523 (see Fig. 24) discloses a sanitary napkin in which an area projected from another area is provided by providing an absorbent body in a concentrated manner in the central portion of a surface which is brought into contact with a body of a wearer as the product improving the adhesion between the sanitary napkin and the body. In this configuration of the sanitary napkin, the projected area is inserted inside the labia so that the adhesion is improved compared to an ordinary sanitary napkin.
However, in the napkin according to Japanese Utility Model Publication No. Hei 5-18523, it is difficult to locate the projected area of the sanitary napkin to a predetermined position. In other words, in the improved sanitary napkin as described above, it is necessary, in regard to the sanitary napkin fixed to the underwear, to bring the projected area of the sanitary napkin to the predetermined position between the labia in the action of wearing underwear although the sanitary napkin is fixed to the underwear and fitted to the body by bringing the fixed napkin to the body together with the underwear. In this action, to bring the projected area of the sanitary napkin to the predetermined position is an indirect action so that the control of the action becomes difficult and the wearing position becomes less precise. Also, the projected area is formed by increasing the thickness of the absorbent body, which may cause a strong foreign feeling to the wearer depending on the thickness.

On the contrary, the above-described interlabial pad can obtain the same adhesion as the sanitary napkin of the related art because of the nature of the product, which is inserted in between the labia unlike the sanitary napkin of the prior art that is provided with the projected area.

However, the interlabial pad has the drawback that it is more difficult to fix it than the sanitary napkin because the interlabial pad is fixed between the labia, where a visual check is difficult if compared to the sanitary napkin. Further, if the interlabial pad is not fixed to the right position, menstrual blood leakage may cause immense damage because the interlabial pad is smaller than the sanitary napkin.

The interlabial pad is fixed between the labia and the adhesion is likely to be affected depending on the anatomical topography of the wearer's labia. So, it is required that the interlabial pad can be fixed appropriately according to individual differences.

The present invention has been made to achieve the task shown above. An object of the present invention is to provide an interlabial pad with more intimate contact to the wearer's body and which is usable irrespective of the individual differences in the wearer's anatomical topography of labia, having a structure that is capable of fixing the pad securely and sanitarily between the labia.

### Disclosure of the Invention

In order to achieve the task shown above, the interlabial pad according to the present invention can be fixed irrespective of the length of a wearer's labia by providing a long convex area on the body-facing surface by folding the top sheet and the absorbent body. Further, the long convex area is deformed in a flexible manner according to the shape of the wearer's labia and the surroundings thereof to be fixed to the inner crotch area of the wearer.

The interlabial pad has a structure such that the pad can be fixed by using a sensitive finger cushion dexterously. Specifically, an interlabial pad with a pocket to which a fingertip is inserted smoothly for fixing to the interlabial space is formed.

More specifically, the present invention provides the following items. (1)An interlabial pad with a size capable of being naturally inserted between female labia, which is formed by bonding a water permeable surface side sheet facing a body side and a water permeable or a water impermeable back side sheet facing a garment side so as to enclose an absorbent body for absorbing body fluid, wherein the interlabial pad comprises a long convex area provided along a longitudinal direction of said surface side sheet so that a substantial center part of said surface side sheet in a lateral direction becomes an apex towards the body side; and an extended area formed by being extended from a bottom of said long convex area in the lateral direction of said surface side sheet.

According to the present invention, the long convex area is provided in an extending manner in the longitudinal direction of the top sheet facing the body side of a wearer. So, when the interlabial pad is fixed, the long convex area is engaged in the interlabial space. In other words, the interlabial pad is not only pinched by the labia but also the long convex area provided on the body side face is inserted near the ostium vaginae in the labia minora at the back of the labia so that the occurrence of gaps between the interlabial pad and labia is suppressed significantly.

In addition, because the extension area is provided along the long convex area, when the interlabial pad is worn, the extension area covers the external genitals. So, even if a large amount of menstrual blood flows rapidly in the lateral direction, the extension area positioned in a direction perpendicular to the external genitals becomes a wall to intercept it. In addition to that, since the boundary between the long convex area and extension area becomes a folded back portion pinching the external genitals when the interlabial pad is fixed, the folded back portion plays a role like a groove to prevent menstrual blood from flowing outside so as to receive the menstrual blood even if the extension area can not absorb all menstrual blood.

As shown above, according to the present invention, menstrual blood leakage from the top sheet is prevented not only in the longitudinal direction but also in the lateral direction. In addition, as shown above, the extension area is deformed so that it covers the external genitals to become a wall to prevent menstrual blood from flowing out into the lateral direction. So, it is desirable that the shape is substantially planate.

In addition, the fact that the long convex area provided on the body face side closely contacts the interlabial space means that the interlabial pad is fixed tightly to the body of the wearer.

Thus, the long convex area provided in the interlabial pad according to the present invention has a leakage prevention function as well as a removal prevention function.
In order to enhance the removal prevention function, an adhesive may be applied on the top sheet of the interlabial pad according to the present invention to further improve the contact with the wearer's body.

Said long convex area is formed by at least an inflection portion provided by bending said surface side sheet and said absorbent body.

In the interlabial pad according to the present invention, since the long convex area provided on the body side face to be fitted in between the labia of the wearer is formed only by folding the top sheet and the absorbent body, it can be transformed readily with external pressure. Specifically, the long convex area substantially maintains the original shape in the region in contact with the wearer's labia. However, the other area is unfolded and flattened out. So, the wearer only has to fix the interlabial pad in between the labia and wear an undergarment such that the long convex area shifts its shape according to the anatomical topography of the wearer's labia due to the external pressure generated between the wearer's labia and the undergarment, etc.

On the other hand, if a ready-made projection is provided on the body side face of the interlabial space, the projection does not always fit to all wearers because there are individual differences in the labia shape. For example, if the length of the wearer's labia is longer than that of the projection, the projection can fill only some part of space between the labia so that other part remains empty without the inserted projection. In this case, gaps between the labia could be larger because the labia majora are kept open by the projection inserted in some space between the labia while other space between the labia is left open.

On the other hand, if the ready-made projection is shorter than the wearer's labia, since the projection is not pinched tightly by the labia, the contact area between the projection and the labia becomes small. In this case, effective menstrual blood absorption by the projection is not much expected.

In this respect, since the long convex area of the interlabial pad of the present invention is formed in such a way that it can be transformed readily according to the anatomical topography of the wearer's labia, the interlabial pad can be fixed closely to the body of the wearer without causing an undesirable gap between the wearer's body and the interlabial pad. It is also possible to adopt such a structure that the top sheet and the absorbent body as well as the back side sheet may be folded together to form the long convex area. Therefore, the whole interlabial pad is bent so that the detailed work to bond the back side sheet to the surface side sheet by adjusting to the apparent length the dimension of the folded top sheet and the absorbent body is omitted resulting in a simplified manufacturing process of the interlabial pad according to the present invention. This has an effect that the interlabial pad can be contained easily in the individual wrapping container since the interlabial pad can be folded along the ridge line of the long convex area as an axis.

As described above, the intervention portion between the labia and other portions are formed by transforming the long convex area, both portions are connected smoothly along the anatomical topography of wearer's labia and its peripherals. Occurrence of a gap between the wearer's inner crotch and the interlabial pad is reduced compared to the case where a ready-made projection is provided by projecting only a part of the body side face since the interlabial space and other portion are integrated.

As described above, according to the present invention, the interlabial pad with high functionality can be realized by such a simple configuration of just folding the interlabial pad. In this respect, since the sanitary napkin is originally thick due to the product structure, if it is folded in the same manner as the interlabial pad according to the present invention, the sanitary napkin has an inappropriate thickness for being pinched in between the labia so as to deteriorate significantly the wear feeling. In other words, the present invention has been accomplished by utilizing the product property of the interlabial pad that is significantly thinner than the sanitary napkin.

According to the present invention, the interlabial pad can have the property to fit the personal labia shape more accurately by using materials that are flexible and free to be transformed.

The interlabial pad according to the present invention can be contained compactly by taking down the long convex area, which is bent to the left or right facing the lateral direction.

The interlabial pad, comprises a restriction mini sheet piece for restricting spreading of said inflection portion, wherein the mini sheet piece is bonded on an opposite side to the body side of said back side sheet.

Since the long convex area shown in (2) above is formed simply by folding the top sheet and the absorbent body, it may happen that the inflection portion is unbent to flatten the long convex area so that the long convex area may not be inserted in between the labia before the wearer puts the interlabial pad in between the labia. In this respect, the restriction mini sheet piece is attached to restrict the expansion of the inflection portion for preventing unnecessary deformation of the long convex area. By this, the prefferred shape of the long convex area can be maintained without specific attention of the wearer before fixing the interlabial pad.

The interlabial pad comprises in said long convex area, a restricted part where spreading of said inflection portion is restricted by said restriction mini sheet piece and an unrestricted part where spreading of said inflection portion is not restricted by said restriction mini sheet piece.

According to the present invention, it is possible to regulate the deformation of the long convex area by changing the position to attach the restriction mini sheet piece. For example, for a user who is likely to have leakage at the front side (clitoral side), it is possible to provide an interlabial sheet whose front side is transformed into a planar shape by providing the restriction mini sheet piece at the rear region. For a user who is likely to have leakage at the rear side (analis side), it is possible to provide an interlabial sheet whose rear side is transformed into a planar shape by providing the restriction mini sheet piece at the front region.

As shown above, according to the present invention, it is possible to provide an interlabial pad having various modes of leakage prevention functions only by changing the position of fixing the restriction mini sheet piece. As a result, a user can select a suitable kind of interlabial pad according to the leakage mode of her menstrual blood.

Said mini sheet piece is provided by being bonded to the opposite side to the body side of said back side sheet in one or more bonding areas of each side in the longitudinal direction of said back side sheet and being non-bonded in one or more non-bonding areas in the lateral direction of said back side sheet; and wherein at least one of said one or more non-bonding areas form a finger insertion opening between said restriction mini sheet piece and said back side sheet.

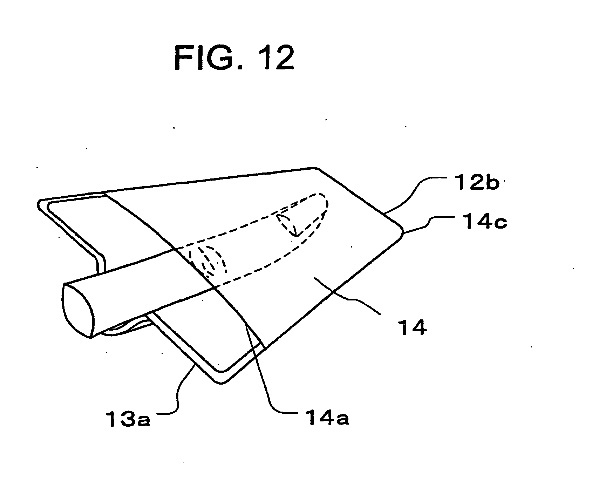

At least one of the both sleeves formed with the restriction mini sheet piece attached to the opposite side to the body side of the interlabial pad in the lateral direction of the back side sheet according to the present invention is not bonded to the back side sheet surface. By this, a sleeve opening between the one sleeve of the restriction mini sheet piece that is not bonded and the back side sheet is formed to be a finger insertion opening in which a finger can be inserted (see Fig.12).

In addition, the restriction mini sheet piece is bonded only to the left and right side of the back side sheet in the longitudinal direction. Inside of the back side sheet is not bonded (not glued). Thus, the restriction mini sheet piece is attached to the back side sheet like standing astride with one leg fixed to one side and the other leg fixed to the other side of the back side sheet so that a space (for finger insertion space) in which a finger is inserted to hold the interlabial pad is formed between the one side and the other side of the back side sheet

Here, if both sleeve portions of the restriction mini sheet piece are non-bonded to the back side sheet in the lateral direction, the finger insertion space becomes a through-hole (in a tunnel state). On the other hand, if one sleeve portion of the restriction mini sheet piece is bonded, the finger insertion space becomes like a cave.

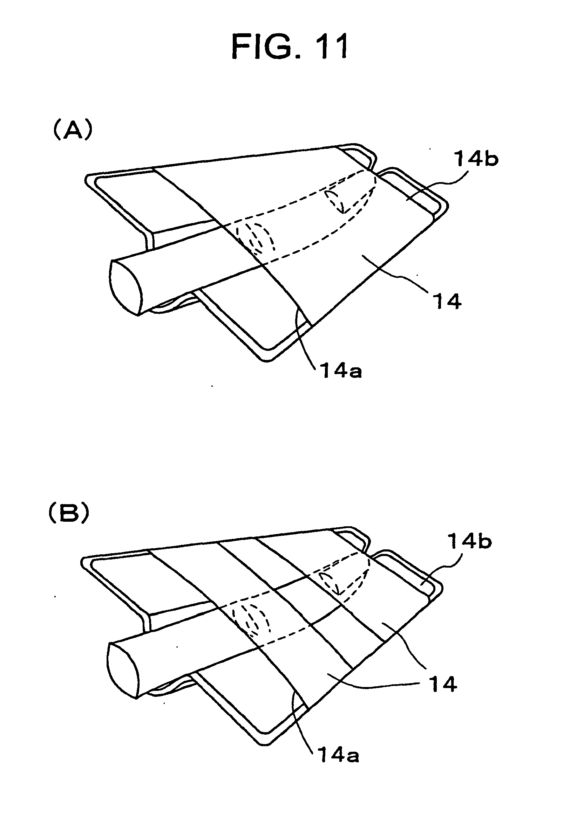

If the restriction mini sheet piece is composed of multiple pieces, the number of bonded sections at left and right sides will increase in the longitudinal direction of the back side sheet as the number of pieces of the restriction mini sheet pieces increases. For example, if the number of the restriction mini sheet pieces is two, two bonded portions are provided for each restriction mini sheet piece (see Fig.11 (B)).

In addition to that, in the present invention, the "side portion" in the longitudinal direction of the back side sheet includes not only the regions corresponding to the edges of the interlabial pad but also the edges and peripherals of the restriction mini sheet piece that can be bonded.

As shown above, according to the present invention, since the restriction mini sheet piece is attached to the back side sheet in such a way that the finger insertion opening and the finger insertion space continued from the opening are formed, it is possible to fix the interlabial pad at the fingertip temporarily by inserting the finger into the finger insertion opening. By this, it is possible to fit the long convex area that is provided on the body face side of the interlabial pad to the appropriate position between the labia, the surroundings of which is hard to be seen, by locating the accurate fixing point.

Here, for example, the fingertip with a flat shape can be inserted in such a way that it faces the surface of the sheet naturally without taking abnormal orientation against the surface of the sheet, by positioning the restriction mini sheet piece near the inflection portion to form the finger insertion opening having an appropriate size for the wearer's fingerbreadth.

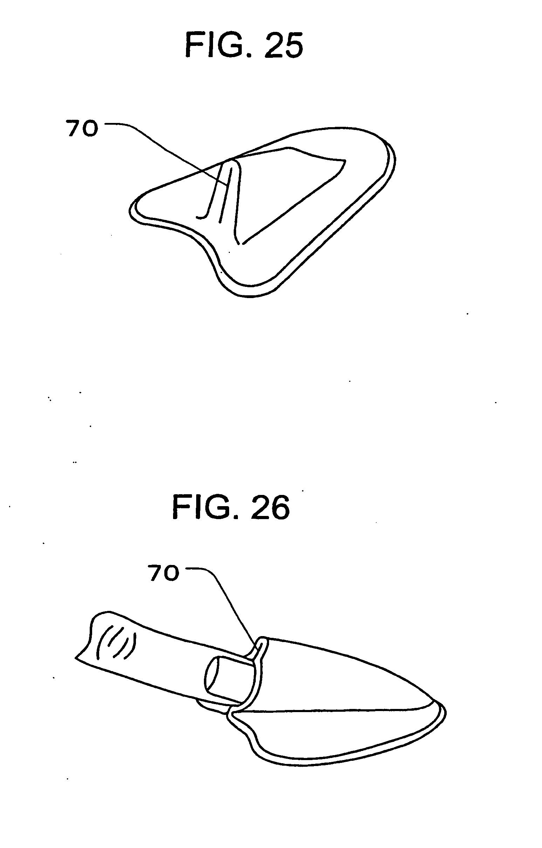

In other words, in this case, the finger breadth opening is formed towards the direction of the back side sheet surface, differently from the incontinence pad of the conventional example (Japanese Patent Publication No. Hei 6-506368), where the finger insertion opening is closed in the ordinary state (see Fig.25), and first, insert the finger in the direction that makes a right angle with the incontinence pad (see Fig.26), then, the finger cushion can face the interlabial pad side only after rotating the finger. In the present invention, the finger breadth opening is formed towards the direction of the back side sheet surface primarily. Thus, when inserting the finger in the finger insertion opening, it is possible to specify the finger insertion direction so that the finger cushion faces the sheet surface side resulting in quicker and more accurate fixing of the interlabial pad.

Further, if the restriction mini sheet piece is attached to the back side sheet in such a way that a pocket is formed, since the back side sheet adheres to the restriction mini sheet piece at the position which the fingertip contacts, it is possible to definitely evade the incident that the fingertip touches menstrual blood so as to fix it in a hygienic condition.

More further, it is also possible to reduce the occurrence of phenomenon such as friction and sticking between the finger tip and the interlabial pad by providing micro concavities and convexities at the position on the back side sheet which the finger cushion touches so as to reduce the contact area between the finger cushion and the opposite side to the body side of the back side sheet. By this, the incident that the interlabial pad is fixed to an unexpected position due to the condition of the wearer's fingertip, for example wet condition, is prevented. It is also possible to pull out the finger smoothly and prevent displacement after fixing.

As used herein, the term "finger breadth" refers to the dimension other than the thickness of the finger. Specifically, it refers to the width of a finger in the direction of nail width. As used herein, the term "opening of finger breadth" refers to an opening having enough size to insert a finger.
(2) The interlabial pad according to wherein the inside of said long convex area forms a long hollow space.
   According to the present invention, since inside of the long convex area is hollow, when an undergarment is worn after fixing the interlabial pad, it is possible to flatten out the long convex area without difficulty by the external pressure caused by the wearer's labia and the undergarment, etc. So, the long convex area is transformed into a shape fitted to the wearer's labia causing further reduced occurrence of the gap between the inner crotch and the interlabial pad.
(3) The interlabial pad according to any one from (1) to (2) wherein the cross section of said long convex area is substantially triangular.
   As described above, by making the long hollow space inside the long convex area a substantially triangular shape, the long hollow space is likely to fit a finger when the finger is inserted into the long hollow space when the interlabial pad is worn. So, the fixing point will be detected more easily since the interlabial pad is fixed securely to the finger inserted into the long convex area.
   When the interlabial pad having the long convex area is fixed, the left and right labia majora and labia minora that are closed under a normal condition are somewhat opened due to the long convex area inserted between the labia. However, according to the present invention, if the cross section of the long convex area is arranged in a substantial triangle so that the apex is positioned at the inner area of the interlabial space, the top sheet can contact any portion in the labia minora. By this, the long convex area, that is, the menstrual blood absorbable portion can be fitted between the labia so that the menstrual blood leakage prevention function as well as the said easy fitting is improved.
(4) The interlabial pad according to any one from (1) to (3) wherein the cross sectional area of said long convex area in the longitudinal direction becomes gradually smaller from one end to another end.
   As described above, the long hollow space is preferably tapered according to the tapered shape of the finger in the case of inserting the finger into the long hollow space inside of the long convex area. In this respect, according to the present invention, since the cross-sectional area of the long convex area gets smaller towards one end, the long hollow space is in close contact with the finger causing more secure fixation and holding of the interlabial pad at the fingertip.
(5) The interlabial pad according to any one from (1) to (4), wherein the cross section of the long convex area is formed in a triangle shape with a height thereof preferably from 5 mm to 30 mm, and more preferably from 10 mm to 20 mm, and with a width of the bottom thereof preferably from 1 mm to 20 mm, and more preferably from 2 mm to 10 mm.
   Statistically, the depth of the female labia is about 17mm. So, if the height of the said substantially triangular shape is larger than 30mm, the hem of the substantial triangle may not be pinched between the labia. So, the existence of the long convex area separates the wearer's inner crotch and the top sheet of the interlabial pad according to the present invention so as to cause gaps between them so that menstrual blood may leak from the gaps. On the other hand, if the height of the substantially triangular shape is less than 5mm, the apex cannot contact the deep bottom of the crevice between the labia and a gap is generated between the ostium vaginae and the apex of the long convex area. It may cause menstrual blood to leak from the gap and that the function to prevent the product from dropping off does not work out completely.
   On the other hand, in the case that the long convex area is formed by folding each sheet or the absorbent body, if the length of the bottom edge of the substantially triangular shape is 20mm or more, the apex with substantially triangular shape is too obtuse and the apex can not reach the vicinity of the ostium vaginae. So, the long convex area cannot be inserted to get near to the ostium vaginae so as to generate a gap and the substantially triangular shape of the cross section of the long convex becomes less meaningful. On the other hand, if the bottom edge is less than 1 mm, the apex becomes too sharp so as to give a strange feeling to the wearer.
   In this respect, the long convex area of the present invention is formed in such a way that the cross section thereof is the substantially triangular shape that is appropriate for fitting in between the wearer's labia. So, adhesion and fixation of the long convex area against the labia are high so that the menstrual blood leakage prevention function and interlabial pad removal prevention function perform effectively.
(6) The interlabial pad according to any one from (1) to (5) wherein a length of said long convex area in the longitudinal direction is preferably 60 mm to 150 mm and more preferably 100 mm to 120 mm.
   The length of the interlabial pad according to the present invention in the longitudinal direction is at least 60mm. So, the interlabial pad can be applied to any wearer irrespective of the length of her labia in spite of individual differences in the labia shape. In other words, since it is designed to be longer than the average length of external genitals, it can be well applied to users having rather long labia. Even for a wearer having rather short labia, since the portion of the long convex area that is not fitted into the labia is flattened out, the length does not make any difficulties in fixing it. However, if the length of the interlabial pad in the longitudinal direction is too long compared to the length of the labia, the wear feeling is deteriorated. So, in the present invention, the length dimension should not exceed 150mm.
(7) The interlabial pad according to any one from (1) to (6) wherein the pad is a sanitary napkin concomitant interlabial pad used together with a sanitary napkin.
   Some women use several napkins layered when the amount of menstrual blood is large. However, there was a disadvantage such as a bulky feeling and an outside appearance change. In addition, since sanitary napkins are layered on areas other than the vicinity of the ostium vaginae and since layered pads are not required at the areas, rash and excessive humidity may be caused. In this respect, with the interlabial pad according to the present invention, sanitary products are layered only on the labia and their vicinity so as to have little effect on the wear feeling and the outside appearance, and in addition, occurrence of excessive humidity and rash around hips can be reduced.
   Thus, according to the present invention, since only the interlabial pad is to be changed without replacing the sanitary napkin, the wearer does not have to carry spare sanitary napkins that have a conspicuously large size.
(8) The interlabial pad according to any one from (1) to (7) wherein said interlabial pad is an interlabial pad for incontinence.
   According to the interlabial pad of the invention, the pad can be used as an incontinence absorbing pad. That is, the ostium vaginae where the blood is discharged and a urethral meatus where urine is discharged locate between the labia, and the interlabial pad according to the present invention to be used between the labia can absorb urine also.
   As described hereinbefore, the pad according to the present invention can absorb urine around the labia, especially around the urethral meatus and is useful as an absorbing pad for incontinence, especially for a light incontinence.
(9) The interlabial pad according to any one from (1) to (7) wherein said interlabial pad is an interlabial pad for absorbing vaginal discharge.
   In accordance with the invention, the interlabial pad can be used as a pad for absorbing vaginal discharge. The interlabial pad is used between labia and can absorb excretions other than menstrual blood from the ostium vaginae ( absorbing vaginal discharge).
   As described above, the pad can absorb vaginal discharge in order to decrease discomfort for the wearer, and is useful also for the user who is not menstruating.
(10) A method for manufacturing the interlabial pad as in claim 10 recited in any one from (1) to (9) the pad comprising an absorbent sheet composed of said surface side sheet, said absorbent body, and said back side sheet; wherein said restriction mini sheet piece has shorter width than that of the absorbent sheet in the lateral direction and is bonded to the absorbent sheet at each side edge of the mini sheet piece and the absorbent sheet such that a sagged portion on a body side of the absorbent sheet is made so as to make the sagged portion form the long convex area.
   According to the present invention, utilizing the product property of the interlabial pad as being flat and thin, it is possible to form the interlabial pad that is rich in fitting with the wearer's inner crotch and that has an enhanced leakage prevention function and removal prevention function by such an easy method of bending or folding the sheet and the absorbent body.
   Because of the simple structure, it is possible to manufacture an interlabial pad having high functionality without making the manufacturing process too complicated and without raising the production cost so much compared to the ordinary interlabial pad.
   Since the restriction mini sheet piece according to the present invention is formed in such a way that the width thereof is shorter than the width of the absorbent layer in the lateral direction, it is possible to provide the corrugated area by bonding the left and right side edges of the absorbent layer sheet corresponding to left and right side edges of the mini sheet piece.
   It is also possible to make the restriction mini sheet piece shrink by itself to generate corrugates after fitting the restriction mini sheet piece in a stretched state in conformity to the width in the lateral direction of the absorbent layer sheet using an extensible material for the restriction mini sheet piece. It eliminates the need for the work of bending the absorbent layer sheet in advance in conformity to the width of the restriction mini sheet piece in order to attach the restriction mini sheet piece resulting in a simplified process of work.
(11) A wrapping body for wrapping said interlabial pad as recited in any one from (1) to (9), wherein said interlabial pad is contained in a wrapping container for individual wrapping.
   According to the present invention, since the interlabial pad is wrapped individually, it is possible to handle it more cleanly and conveniently than the case where a plurality of interlabial pads are wrapped in a single wrapping container.
(12) The wrapping body for wrapping said interlabial pad as recited in (11) in a wrapping container for individual wrapping, wherein said interlabial pad is wrapped in the direction orthogonal to said wrapping container.
   According to the present invention, since the interlabial pad is wrapped in a different direction in the wrapping container, the wrapping body can be formed in such a way that the finger insertion opening formed with the restriction mini sheet piece is opened towards the wearer when the wrapping container is unwrapped so as to make easy insertion of the wearer's finger because the direction of unwrapping and finger inserting is aligned.

### Brief Description of the Drawings

Fig.1 is a simplified perspective view showing the body side of the interlabial pad according to this embodiment.
Fig.2 is a simplified perspective view showing the opposite side to the body side of the interlabial pad.
Fig. 3 is a cross sectional view of Fig.1 showing the interlabial pad according to this embodiment.
Fig.4 is a schematic diagram of the length dimension in the lateral direction of the interlabial pad.
Fig.5 is a schematic diagram of the interlabial pad according to this embodiment showing that the cross-sectional areas of the hollow portion at both ends of the long convex area are different.
Fig.6 is a schematic diagram showing the state where a forefinger is inserted into the hollow portion when using the interlabial pad according to this embodiment.
Fig.7 is a drawing showing a way that the interlabial pad according to this embodiment is fitted in between the labia.
Fig.8 is a drawing showing another example of the interlabial pad according to this embodiment.
Fig.9 is a drawing showing the state that the long convex area of the interlabial pad according to this embodiment is deformed.
Fig.10 is a cross section diagram of the long convex area of the interlabial pad according to this embodiment.
Fig.11 is a drawing showing the positions of the non-bonded sections on the back side sheet when a plurality of restriction mini sheet pieces are attached to the interlabial pad according to this embodiment.
Fig. 12 is a schematic diagram showing the state that the one end of the restriction mini sheet piece attached to the interlabial pad according to this embodiment is bonded to the back side sheet.
Fig. 13 is a drawing showing the state that the restriction mini sheet piece of the interlabial pad according to this embodiment is sagged towards the opposite side to the body.
Fig.14 is a schematic diagram showing the state that the interlabial pad according to this embodiment is removed by pulling the restriction mini sheet piece.
Fig. 15 is a cross section diagram of the cross section of the interlabial pad in the lateral direction in order to explain the bonding state of the restriction mini sheet piece.
Fig.16 is a drawing showing the state of the test to measure peel strength of the fastening tape.
Fig. 17 is a drawing showing the state of the test to measure shear strength of the fastening tape.
Fig.18 is a drawing showing the state that the interlabial pad according to this embodiment is used with a sanitary napkin.
Fig. 19 is a drawing showing the state that the interlabial pad according to this embodiment is folded to be contained in the wrapping container.
Fig.20 is a schematic diagram showing the state that the interlabial pad having the ready-made projection is fixed.
Fig. 21 is a schematic diagram showing the state that the interlabial pad according to this embodiment without the restriction mini sheet piece at the rear end of the back side sheet is fixed.
Fig. 22 is a schematic diagram showing the state that the interlabial pad according to this embodiment with the restriction mini sheet piece at the rear end of the back side sheet is fixed.
Fig.23 is a schematic diagram showing the state that the extension area provided in the interlabial pad according to this embodiment covers the external genitals.
Fig.24 is a schematic diagram showing the ordinary state of a conventional prior art of a sanitary napkin having the ready-made projection area.
Fig.25 is a schematic diagram showing the ordinary state of a conventional prior art of an incontinence pad having the finger insertion opening.
Fig.26 is a schematic diagram showing the state of inserting a finger in a conventional prior art of an incontinence pad having the finger insertion opening.

### Best Mode of Carrying Out the Invention

By referring to the accompanying drawings, the interlabial pad according to this embodiment will be explained.

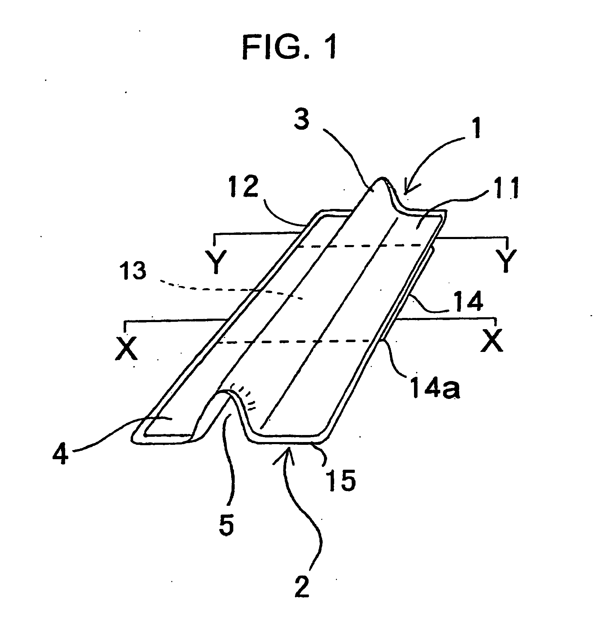

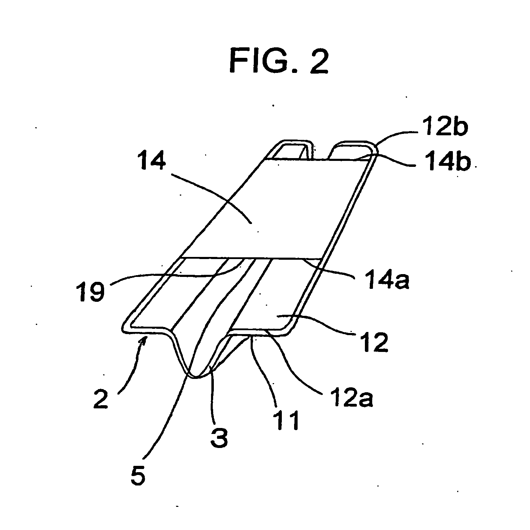

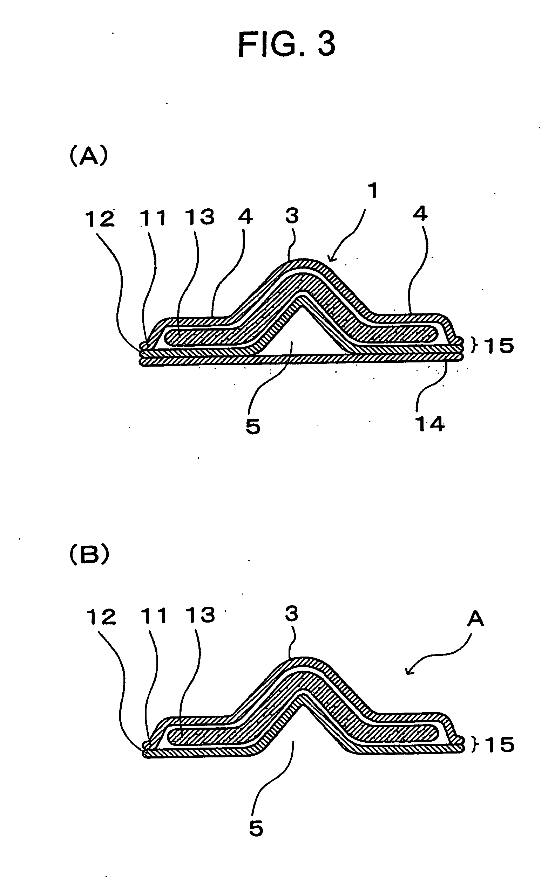

Fig.1 is a simplified perspective view showing the body-facing side of the interlabial pad according to this embodiment. Fig.2 is a .simplified perspective view showing the opposite side to the body side of the interlabial pad. Fig.3 is a cross sectional view of Fig.1 showing the interlabial pad according to this embodiment.

### [Basic structure]

As shown in Fig. 1, the interlabial pad according to this embodiment 1 is comprised of the absorbent layer sheet 2 composed of the top sheet 11 made of a liquid permeable material, the back side sheet 12 made of a liquid impermeable material and the absorbent body 13, and the restriction mini sheet piece 14 made of a liquid impermeable material.

The long convex area 3 composed of the corrugated area generated by folding the absorbent layer sheet 2 is provided in the approximate center of the lateral axis along the longitudinal axis of the absorbent layer sheet 2. The extension area 4 is provided on the skirt of the long convex area 3. On the other hand, as shown in Fig. 2, the restriction mini sheet piece 14 is attached to the opposite side to the body side of the absorbent layer sheet 2 in such a way that the broadening of the long convex area 3 is partially restricted.

As shown in Fig.3(A) and Fig.3(B), the absorbent layer sheet 2 is formed integrally by bonding the top sheet 11 and the back side sheet 12 which are bonded to each other at the marginal area 15 in such a way the absorbent body 13 is enclosed.

In a preferred embodiment, the top sheet 11 and the back side sheet 12 are bonded to each other at the marginal area 15. In that case, it is desirable that the absorbent body 13 is not pinched by the marginal area 15 for bonding the top sheet 11 and the back side sheet 12. For that purpose, for example, the absorbent body 13 should be enclosed in the pouched area formed with the marginal area 15 after bonding only the top sheet 11 and the back side sheet 12. With regard to this, if the absorbent body 13 is pinched to be bonded to the marginal area 15, the marginal area 15 becomes hard. By the method shown above, however, hardening of the marginal area 15 can be evaded resulting in a more preferable wear feeling. The dimension of the absorbent body 13 may be the same as that of the interlabial pad 1. It also may be a dimension reduced in advance in such a way that a distance of 2 to 10mm can be provided from the outer border of the interlabial pad so that the absorbent body should not be pinched between the marginal area 15.

The top sheet 11 and the back side sheet 12 are bonded to each other with heat embossing and/or hot melt type adhesives. The absorbent body 13 is bonded to the top sheet 11 and the back side sheet 12 in order to prevent the interlayer separation against the sheet 11 and 12.

### [The restriction mini sheet piece]

In this embodiment, the restriction mini sheet piece 14 is, fitted as shown in Fig. 2, so that the hollow portion 5 is formed in such a way that a finger can be inserted between the restriction mini sheet piece 14 and the back side sheet 12. The one end 14a of the restriction mini sheet piece 14 is not bonded to the back side sheet 12 so that the finger insertion opening 19 is formed. The fixing position of the restriction mini sheet piece 14 on the back side sheet 12 is provided near the rear end 12b to be positioned at the backward side when wearing.
By this, the area of the fingerprint side of the finger inserted from the finger insertion opening 19 to the hollow portion 5 touching the back side sheet 12 is larger so as to increase sense of unity between the interlabial pad 1 and the fingertip compared to the case where the fixing position is provided near the front end 12 to be the front (clitoral side) or middle portion of the sheet.

Further, as apparent from Fig.2, the restriction mini sheet piece 14 does not exist at the rear end 12b of the back side sheet 12. So, as shown in Fig. 3(A), the restriction mini sheet piece 14 exists in X-X view of Fig.1, however, it does not exist in Y-Y view of Fig. 1 as shown in Fig.3(B).

Here, if the end section 14b of the restriction mini sheet piece 14 is bonded to the end section 12b of the back side sheet 12 to form the pocket like finger insertion space, as shown in Fig.12 below, Y-Y view of Fig. 1 will be the same as Fig.3 (A).

The longitudinal dimension of the restriction mini sheet piece 14 may be smaller than the back side sheet 12 according to this embodiment. It is also possible to facilitate positioning the back side sheet 12 and the restriction mini sheet piece 14 when they are bonded by making the longitudinal dimension of the restriction mini sheet piece 14 same as the back side sheet 12.

As used herein, the term "pocket-like" refers to a pouched member in which a finger can be inserted completely without a hindrance. Its cross section is preferably planiform or the like. However, it may be in many other forms such as conical shape, and is not particularly limited.

### [Size of the interlabial pad]

The length dimension of the interlabial pad 1 according to the present invention is preferably 60 to 150mm, more preferably 100 to 150 mm in the longitudinal direction. With regard to this, if it is longer than 150mm, the contact area between an undergarment, etc. and the opposite side face to the body side of the interlabiale pad becomes too large so that it may be caused that the interlabial pad falls off due to the friction force being stronger than the holding force of the interlabial pad between the labia.

In the meantime, if it is less than 60mm, the dimension of the long convex area 3 formed on the body side face is not sufficient for the length of all wearers' labia. In this respect, it is possible to provide the interlabial pad that is preferable to wear and have enough function for preventing leakage by the dimension in the range shown above.

Apparent length dimension of the interlabial pad in the lateral direction is preferably 10 to 60mm, and more preferably 20 to 40mm. If the length dimension in the lateral direction is longer than 60mm, the interlabial pad may fall off since the area not pinched by the labia is rubbed with thighs of the wearer and since the friction generated due to this exceeds the retaining force between the labia. On the contrary, if the length dimension in the lateral direction is smaller than 10mm, the area that lies between the labia decreases and the contact area with the inner face of the labia is reduced to cause higher risk in falling off the interlabial pad since the area to be pinched between the labia becomes smaller.

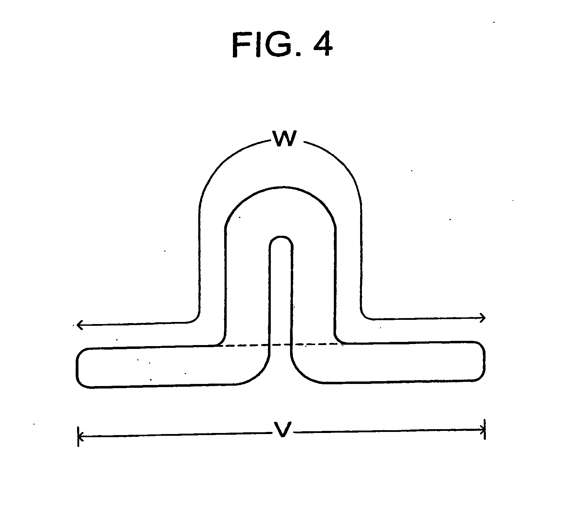

As used herein, the term "apparent length" refers to the distance between two points having the smallest length dimension (falls under V in Fig.4). This is a deliberate definition considering the case where the distance between two points in a flattened pad from a three dimensional shape is sometimes used as an actual distance (falls under W in Fig. 4) in relation to manufacturing process.

In addition, in this embodiment, the length of the long convex area 3 is about 120mm. So, there is little possibility that the length of the wearer's labia is larger than that of the product and leakage of the body fluid along the labia in the longitudinal direction can be reduced. The long convex area 3 that is not intervened by the labia may not cause any disadvantages as shown below, since it is deformed into a flat shape when it is worn.

The thickness of the interlabial pad according to the present invention is preferably 0.5 to 20mm, and more preferably 2 to 0mm. Since the interlabial pad is fixed into the sensitive interlabial space, if the thickness is 20mm or more, the wearer feels foreign-body sensation. On the other hand, if it is 0.5mm or less, the volume of the absorbent body contained is likely to be insufficient for absorbing menstrual blood and there is the possibility that menstrual blood seeps from the interlabial pad. In this respect, such problems can be evaded by the thickness shown above.

Especially, in this embodiment, the thickness of the absorbent layer sheet 2 itself has considerable influence upon the wear feeling since the long convex area 3 is formed by folding the absorbent layer sheet 2. Preferred wear feeling can be obtained by the thickness within the dimensional range shown above.

### [Materials]

Materials usable for the top sheet 11 are not particularly limited so long as they have a liquid permeable structure such as fabrics, nonwoven fabrics, porous plastic sheets. Specifically, the materials usable herein include a porous film where a thermoplastic film is perforated by heat embossment, mechanical processing, etc., and a compound sheet of a porous film and a nonwoven sheet. Besides that, materials shown below are also usable.

Hydrophobic synthetic fibers usable herein include mono-fibers of PE (polyethylene), PP (polypropylene) or PET (polyethylene terephthalate); fibers of a graft polymer of PE and PP; or conjugated synthetic fibers of a core-sheath structure having a core of PP or PET and a sheath of PE. Woven and nonwoven webs usable herein include natural fibers such as cotton, silk, hemp; recycled fibers such as recycled cellulose fibers, for example rayon, acetate fibers, etc; synthetic fibers such as polyolefin fibers, polyacrylonitrile fibers, polyester fibers, polyamide fibers, polyvinyl alcohol fibers, polyurethane, nylon. In the case of nonwoven webs, web forming methods may be selected from dry methods (card method, spunbond, meltblown, airlaid), wet methods and combination thereof. Bonding methods include spunlace, thermal bonding, needle punch, etc. taking advantage of the columnar flow.

Among these materials, considering liquid mobility from the inner face of the labia, chemical stimulation due to activators and the adhesiveness to the inner face of the labia, a spun lace nonwoven fabric wherein rayon having fineness of 1.1 to 4.4 dtex, fiber length 7 to 51mm and being 40 to 80% on the basis of total specific weight per unit area is laminated by on the body face side, rayon having fineness of 1.1 to 4.4 dtex, fiber length 7 to 51 mm and being 14 to 42% on the basis of total specific weight per unit area, and PET having fineness of 1.1 to 4.4 dtex, fiber length 7 to 51 mm and being 6 to 18% on the basis of total specific weight per unit area are mixed to be laminated on the garment face side, and two layers are laminated in such a way that their total specific weight per unit area is 20 to 60 g/m², then, fibers are entangled with one another by water flow entanglement and dried, the thickness thereof is adjusted in the range from 0.13 to 0.50mm is preferable. In this case, by mixing PET on the garment face side, adhesion to the inner wall of the labia is secured since it is possible to maintain bulkiness even if the water permeable sheet is in wet condition.

Perforated plastic sheets usable herein include perforated sheets of thermoplastic resins, such as PE (polyethylene), PP (polypropylene) or PET (polyethylene terephthalate) and porous foaming materials. If required, a filler of 0.5 to 10% by weight composed of titanium oxide, calcium carbonate, etc. is preferably mixed to make the sheet clouded. A perforated film perforated by perforation, heat embossing, mechanical processing, etc., can be used. Further, a conjugated sheet of a perforated film and a nonwoven fabric may be also used.

### <Absorbent body>

Materials to be used for the absorbent body contained in the interlabiale pad include pulp, chemical pulp, rayon, acetate, natural cotton, water-absorbent polymer, fibrous water-absorbent polymer, synthetic fiber and mixture thereof. A mixture blended as required is made into a sheet by technologies such as pressure bonding by embossing process, entanglement by needling that are well known in the art. The sheet may be adjusted by bulking, layering, folding, etc. as required.

Materials for the sheet may be used in a sheet or powdered. Their types of usage are not particularly limited. Absorbent bodies can be used so far as they can absorb and retain liquid (body fluid). It is desirable that they have bulkiness, are barely deformed, have less chemical stimulus, and further have flexibility to well fit to the labia. Specifically, a nonwoven sheet made by dot pattern embossing that is adjusted to bulkiness of 2 to 10mm, preferably 3 to 5mm wherein a pulp selected from the range of fiber length from 1 to 10mm is layered by 50 to 150g/m² on the garment face side, and a rayon having mixing ratio of 60 to 90% selected from the range of fineness from 1.1 to 4.4dtex and fiber length 20 to 51 mm and a natural cotton having mixing ratio of 40 to 10% are layered by 150 to 250g/m² on the body face side. By this, liquid is easily moved from the body face side to the garment face side and absorbing/retaining ability is improved. Further, by providing a mesh spun lace nonwoven fabric composed of a rayon having fineness of 1.1 to 4.4 dtex, fiber length 25 to 51 mm with a specific weight per unit adjusted from 15 to 40g/m² on the body-facing side of the said pulp layer, the liquid moved from the body-facing side is dispersed by the mesh spun lace to be guided to almost all areas of the pulp layer so that larger quantity of liquid can be absorbed effectively.

### <Back side sheet>

Materials usable for the back side sheet 12 are not particularly limited so far as they are sheet like structural substances such as woven fabrics, nonwoven fabrics, plastics. Examples thereof include, in the case of liquid impermeable materials, liquid impermeable films mainly composed of PE, PP, etc., air-permeable resin films, nonwoven fabrics of spunbond, spunlace, etc. where a resin film having superior air-permeability is bonded to the backside. Considering flexibility that does not deteriorate wear feeling, films composed mainly of low density polyethylene (LDPE) having specific weight per unit area of 15 to 30g/m² are generally used. More preferably, the films may be provided with convex projections by embossing process to lower the contact ratio and friction resistance in order to evade the risk that the small sized pad is removed from the labia due to a large friction if the water impermeable sheets contact with each other, with a pad, with an undergarment used at the same time when the sheet is worn between the labia.

It is possible to prevent menstrual blood retained in the absorbent body 13 from oozing out of the interlabial pad by using water impermeable materials for the back side sheet 12. And it is also possible to reduce humidity and unpleasantness of the wearer who uses the interlabial pad by using moisture permeable materials for the back side sheet 12.

### <Restriction mini sheet piece>

Materials to be used for the restriction mini sheet piece 14 are preferably selected considering the strength with which the sheet may not be damaged when inserting a finger. They can be selected from nonwoven fabric sheets, elastic stretch nonwoven fabrics, films, form films, elastic stretch films foaming sheets, tissue paper and laminated materials thereof without particular limitation. Specifically, it is possible to select a film composed mainly of an LDPE resin having thickness of 15 to 30µm. The restriction mini sheet piece 14 can be adjusted by the methods such as coloring, printing of patterns so that it has a color tone, a pattern and a chroma that are different from the back side sheet of the interlabial pad with which the wearer can identify the restriction mini sheet piece 14 easily.

It is also effective to apply extensity or elasticity to the restriction mini sheet piece 14 in the lateral direction of the back side sheet 12 in order to use the interlabial pad of the present invention irrespective of the size of the wearer's fingertip.

It is possible to use an extensible spun bond nonwoven fabric having a stress of 0.1 to 0.5 N/25mm when extended by 5% at constant speed extension with gripping interval of 100mm, pulling speed of 100mm/min in order to apply extensity to the restriction mini sheet piece 14. It is also possible to use a fibrous sheet and a film sheet using a thermoplastic elastomeric resin; a single elastic and stretchy material such as elastomeric resin, natural rubber, and a mixture thereof with an inelastic material.

The interlabial pad 1 of the present invention may be composed of biodegradable materials and/or water dispersible materials and/or water soluble materials. These kinds of interlabial pads can be flushed in a lavatory bowl as they are after use since they are decomposed naturally with age or actively. So, the used interlabial pad 1 can be disposed easily and cleanly. In addition, wastes in bath rooms can be reduced. In other words, the wearer has only to stand with her legs apart before a toilet bowl and let down the interlabial pad 1 from the labia in order to discard the pad. The wearer is relieved of the troublesome task to discard used products by hand. In addition, there is an advantage that wastes in a bath room can be reduced.

The wrapping container also can be flushed down in a toilet by constructing the individual wrapping container to contain the interlabial pad 1 of the present invention with biodegradable materials and/or water dispersible materials and/or water soluble materials. By this, the wearer is relieved of the troublesome task to discard the used wrapping container and wastes in a bath room can be further reduced.

In this Specification, "biodegradability" means that a substance is decomposed into gas such as carbon dioxide or methane, water, and biomass under anaerobic or aerobic condition according to the natural process under the existence of bacteria represented by actinomycetes and other microbes, and also means that the biodegradability (biodegradable rate and biodegradable degree) of the substance equals to a material naturally generated such as fallen leaves or a synthetic polymer generally recognized having the same biodegradability under the same environment.

"Water dispersibility" is synonymous with "water collapsibility" and refers to characteristics that fibers are capable of dispersing in sufficient fineness in a large amount of water or water flow in such a way that they at least do not cause any clogging in general toilet piping though limited amount of moisture (menstrual blood) during use does not have effect on them. "Water solubility" refers to characteristics that a substance is dissolved in a large quantity of water or water flow though limited amount of moisture (menstrual blood) does not have effect on it.

The materials and constructions can be used without particular limitation so long as they meet the requirements shown above. Fibers usable for liquid permeable materials include natural fibers and /or chemical fibers. Natural fibers usable herein include tissues, fluff pulps, airlaid pulps chemically bonded with a water-soluble resin, cotton, etc. Hydrophilic fibers usable herein include rayon, fibrilrayon, etc. that are recycled cellulose. Synthetic fibers usable herein include polyester, polypropylene, polyethylene, synthetic fibers of ethylene-vinyl acetate copolymers, etc. processed into hydrophilic, etc. Synthetic biodegradable fibers usable herein include polylactic acids, polybutylene succinates, etc. Water-soluble materials usable herein include carboxymethyl cellulose, polyvinyl alcohol, polyacrylonitrile, etc. Above all, natural fibers such as pulps, cotton, fibers having biodegradability such as rayon, polylactic acids are preferably used. They may be used alone or mixed in the required ratio to form a web or a nonwoven fabric. Web forming methods for synthetic biodegradable fibers such as polylactic acids, polybutylene succinates may be selected from dry methods or wet methods, for example, card method, spun bond, melt blown, airlaid. The method may be selected from a combination of the above.

Bonding methods include spunlace, thermal bonding, needle punch, chemical bonding, etc. Examples of forming methods having water dispersibility include water soluble paper formed into a sheet by using hydrogen bonding of fibers, by entangling fibers, etc.

The fiber length is preferably 2 to 51mm, more preferably 2 to 10mm in order to give favorable water dispersibility. Further, the fineness (gauge) is preferably 1.1 to 4.4dtex in order to have water dispersibility and strength with which materials are not broken when the pad is used.

The total specific weight per unit area of the liquid permeable material is preferably from 20 to 60g/m². The breaking strength of the liquid permeable material is at least 800m N/25mm in lengthwise and breadthwise (breaking strength at constant pulling speed of 100mm/min and knob distance 100mm), more preferably 1000 to 2000m N/25mm considering flexibility when wearing.

A specific example of the liquid permeable material is a wet spun lace nonwoven fabric wherein a rayon fiber having fineness of 1.1 to 4.4dtex and fiber length of 5 to 10mm and a wood pulp are mixed by blend ratio of 90:10 to 70:30 by weight, adjusted to total specific weight per unit area of 25 to 40g/m² and thickness of 0.2 to 0.5mm. The permeable material may be provided with multiple holes. In this case, hole size is from 0.5 to 1.5mm, hole area rate (rate of hole area per unit area) is 3 to 20% to form the material.

It is possible to use natural fibers and/or chemical fibers for the absorbent body 13. Natural fibers usable herein include tissues, fluff pulps, airlaid pulps, chemically bonded with a water-soluble resin, cotton, etc. Hydrophilic fibers usable herein include rayon, fibrilrayon, etc. that are recycled cellulose. Hydrophilic synthetic fibers usable herein include polyester, polypropylene, polyethylene, synthetic fibers of ethylene-vinyl acetate copolymers, etc. processed into hydrophilic, etc. Synthetic biodegradable fibers usable herein include polylactic acids, polybutylene succinates, etc. Water-soluble materials usable herein include carboxymethyl cellulose, polyvinyl alcohol, polyacrylonitrile, etc. Above all, natural fibers such as pulps, cotton and fibers having biodegradability such as rayon, polylactic acids are preferably used. They may be used alone or mixed in the required ratio to form a web or a nonwoven fabric. Further, they can be used with super absorbent polymer such as sodium alginate, farina, starch, carboxymethyl cellulose fabricated into granular or fibrous shape in required blending ratio.

A specific example of the absorbent body 13 is that a wood pulp is laminated to have a specific weight per unit area of 150 to 500g/m² and sealed in tissue, adjusted to thickness of 2 to 10mm. It is also possible to improve menstrual blood absorption and retention capacity by blending the absorbent bodies such as starch by 5 to 30g/m² with the said absorbent body.

Examples of liquid impermeable materials having biodegradability and /or water solubility include: cellulosics such as methyl cellulose, hydroxyethyl cellulose, carboxymethyl cellulose; water-soluble polymers such as polyvinyl alcohol, sodium alginate, sodium polyacrylic acid, polyacrylic ether, polyvinyl pyrrolidone, and isobutylene-maleic anhydride; polylactic acids; polybutylene succinates; starch; dextrin, etc.

They may be alone or blended in required ratio to be formed into a film sheet. Further, water-repellent materials such as silicon may be applied to or blended into the film sheet. They may be laminated on a nonwoven fabric to form a material.

A specific example of the sheet is a film of polyvinyl alcohol having a specific weight per unit area of 20 to 50g/m² wherein at least one side, more preferably both sides of the film are coated with silicon or fluorine by 0.5 to 5µm.

Materials preferably usable for the restriction mini sheet piece 14 include polyvinyl alcohol film and a laminated material of polyvinyl alcohol and tissue, etc.

Bonding methods usable for the present invention include: bonding with polyvinyl alcohol, etc. with water solubility or water swellingness, heat seal bonding, heat seal using a thermoplastic water-dispersible adhesive, hydrogen bonding, etc.

Concrete examples of the wrapping container of water-soluble materials or water-dispersible materials include: a composite material where tissue paper having a specific weight per unit area of 15 to 40g/m² and polyvinyl alcohol having a specific weight per unit area of 20 to 50 g/m² are laminated and silicon is applied to the polyvinyl alcohol side by 0.5 to 1 µm; a spun bond nonwoven fabric composed mainly of a polylactic acid fiber having a specific weight per unit area of 15 to 40g/m², etc.

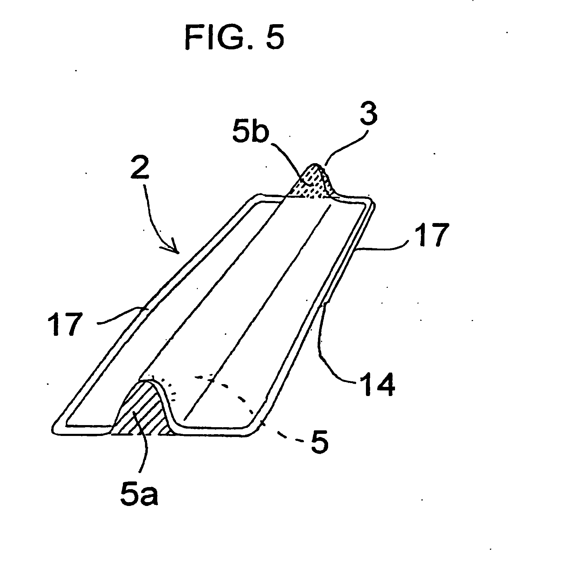

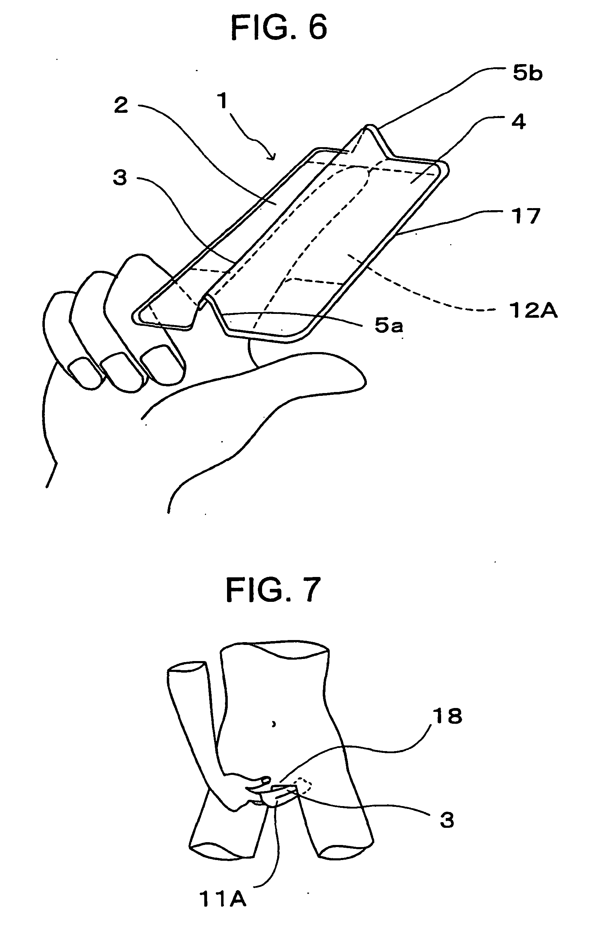

Fig.5 is a schematic diagram showing that the cross-sectional areas at both ends of the hollow portion 5 of the long convex area 3 are different. In this embodiment, the cross-sectional area of the hollow portion 5 of the long convex area is largest at the cross section 5a of the front side. The area decreases gradually towards the cross section 5b at the rear side. So, when using the interlabial pad 1 of this embodiment, as shown in Fig.6, the interlabial pad 1 is held securely with the fingertip by inserting the ball of the forefinger or the middle finger from the cross section 5a to the cross section 5b making it touch the opposite side to the body side 12A of the back side sheet 12.

By inserting the finger in the method shown above, the fingerprint side of the first articulate joint having many receptors touches the opposite side to the body side 12A of the back side sheet 12. So, when guiding the interlabial pad 1 to the labia, as shown in Fig.7, it is possible to reliably guide the long convex area 3 formed on the body face side of the interlabial pad 1 into the back of the interlabial space that can be rarely observed, by making the body face side 11A of the top sheet 11 touch the labia 18 and detecting concavities and convexities of the labia 18 with the sensitive fingertip.

### [Shape of the interlabial pad]

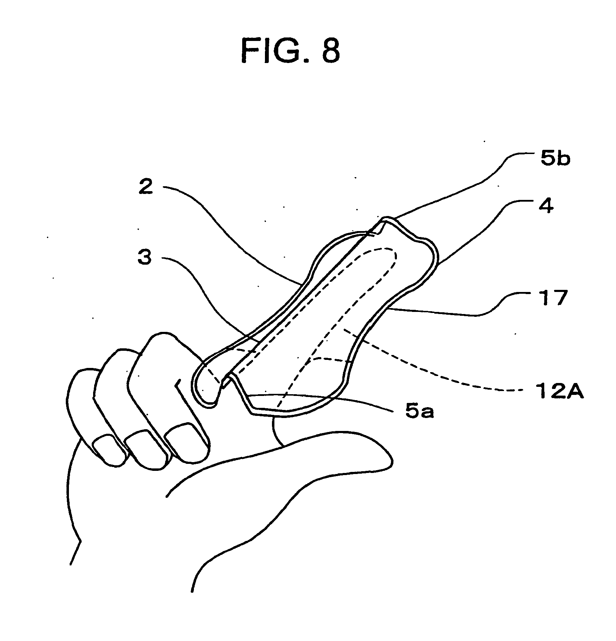

The shape of the interlabial pad 1 of this embodiment should be appropriate for fitting the interlabial space. It may be oval-shaped, egg-shaped, gourd-shaped, drop-shaped, etc. It can fit both the intedabial space and the finger by adopting a shape shown in Fig.8.

Each sheet composing the interlabial pad according to this embodiment is constituted of a flexible sheet and is deformed freely in an elastic manner by outer pressure. So, the shape of the interlabial pad 1 may be folded, twisted in appearance. However, it shows the shape shown above if each sheet is relieved of deformation and removed.

The cross-sectional shape in the lateral direction of the hollow portion 5 is substantially triangular when each side is extended. However, it may be deformed into an oval shape or a circular shape according to the shape of the finger inserted, or it may be deformed to the state where a side leans towards neighboring sides facing the inner side of the hollow portion 5 before use. However, the shape of the cross section still returns to the original shape of the present invention described as above if each side is extended to the specified shape.

### [Deformation state of the interlabial pad]

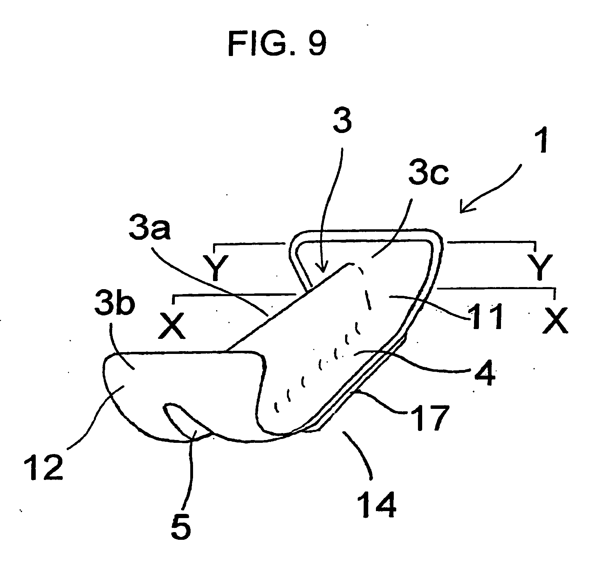

The deformation state of the interlabial pad 1 of this embodiment when worn will now be explained. Fig. 9 is a drawing showing the state that the long convex area of the interlabial pad is deformed.

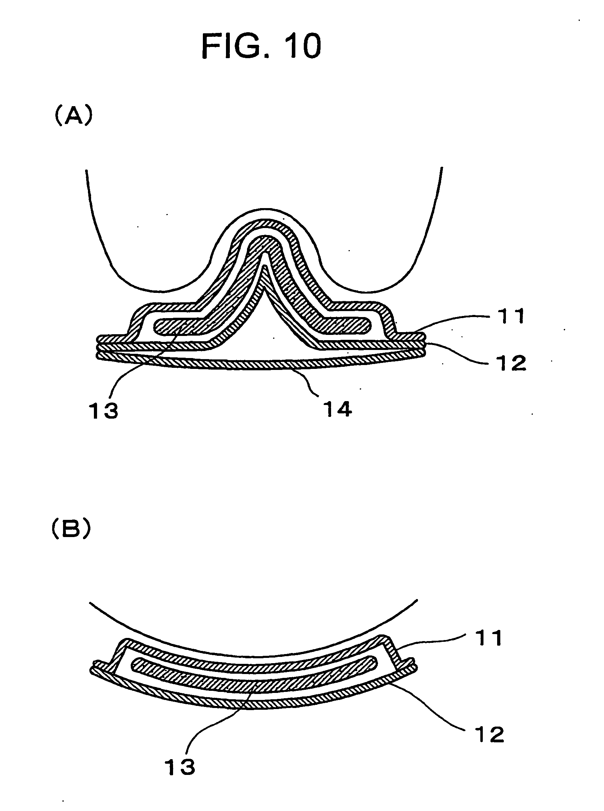

Fig. 10(A) is a cross section diagram showing X-X view of Fig. 9. Fig.10(B)is a cross section diagram showing Y-Y view of Fig. 9.

As described above, since the inner side of the long convex area 3 is hollow portion 5, when the user wears undergarments, etc. after wearing the interlabial pad 1, as shown in Fig. 9, the long convex area 3 maintains the original shape in the non-deformed area 3a that is a part thereof and is deformed into a substantially flat shape in the deformed area 3b and 3c. In other words, as shown in Fig.10 (A), the non-deformed area 3a enters the back of the interlabial space with the shape unchanged. The deformed area 3b and 3c, as shown in Fig. 10(B), are deformed into a substantially flat shape to contact peripheral parts in front and back of the external genitals. Thus, propagation of menstrual blood is almost blocked since the long convex area 3 touches the wearer's labia and is deformed accordingly.

In addition, since the extension area 4 neighboring to the long convex area 3 is fitted in such a manner that it covers the pudenda, it is positioned substantially perpendicular to the body fluid that flows towards the inner wall of the external genitals with high speed in large quantity. Therefore it prevents leakage from the lateral direction of the interlabial pad.

### [Bonding of the restriction mini sheet piece]

Bonding of the restriction mini sheet piece of the interlabial pad according to this embodiment will now be explained. Fig.11 is a drawing showing the state that the restriction mini sheet piece is bonded to the back side sheet in the longitudinal direction. Fig.12 is a drawing showing the state that the restriction mini sheet piece is bonded to the back side sheet in the longitudinal direction.

First, the restriction mini sheet piece 14 bonded to the back side sheet 13 in the longitudinal direction will be explained. In the longitudinal direction of the back side sheet 13 as shown in Fig. 11 (A), there is the second non-bonded section 14b other than the non-bonded section 14a as a non-bonded portion between the restriction mini sheet piece 14 and the back side sheet 12, to be the finger insertion opening. The incident that the fingertip touches menstrual blood reserved on the labia can be prevented by positioning the non-bonded section 14b in such a way that the fingertip is covered with the restriction mini sheet piece 14 when the wearer inserts the finger into the hollow portion 5.

The number of the restriction mini sheet piece 14 may be two or more if the non-bonded section 14b at the end of the pad is positioned in such a way that the wearer's fingertip is covered with the restriction mini sheet piece 14 as shown in Fig.11(B).

In addition to that, as shown in Fig. 12, it is possible to securely evade the incident that the fingertip touches the peripheral area of the contact point accidentally by positioning the one end of the restriction mini sheet piece 14 as the bonded section 14c at the end section 12b of the back side sheet and making the finger insertion space formed between the back side sheet 11 and the restriction mini sheet piece 14 pocket shaped resulting in more hygienic use.

It is possible to restrict broadening of the flexion area of the absorbent layer sheet 2 by bonding the restriction mini sheet piece 14 to the back side sheet 12 at the position corresponding to the non-deformed area 3a of the long convex area 3. By this, it is possible to prevent the long convex area 3 from deforming into a flat shape near the ostium vagina. In addition, since the restriction mini sheet piece 14 is bonded to the back side sheet 12 only at the bonded section 17 and the area inside the bonded section is hollow, the restriction mini sheet piece 14 does not unnecessarily restrict slight deformation in the vertical direction of the non-deformed area 3a in the long convex area 3.

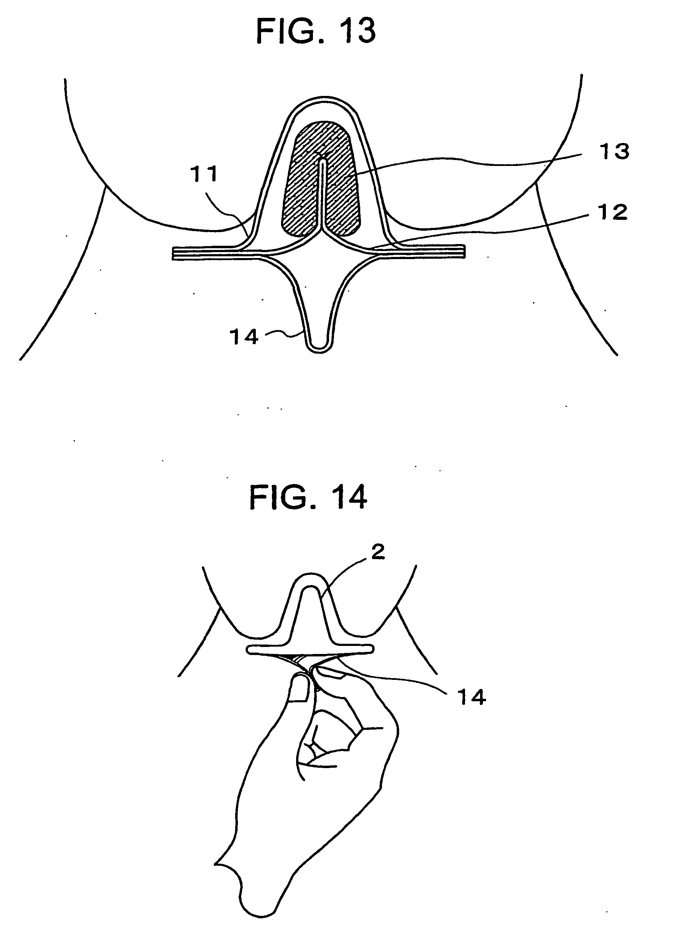

Since the inner side of the restriction mini sheet piece 14 is not bonded to the back side sheet 12 so that the finger insertion space is formed, it is relaxed towards the opposite side face to the body side as shown in Fig.13 after the interlabial pad 1 is fixed and the finger is pulled out. So, it is possible to pull the restriction mini sheet piece 14 in the relaxed state when removing the used interlabial pad 1 as shown in Fig.14. In addition, the wearer can remove the interlabial pad by pinching the restriction mini sheet piece 14 without soiling the fingers by using moisture permeable materials or liquid impermeable materials for the restriction mini sheet piece 14.

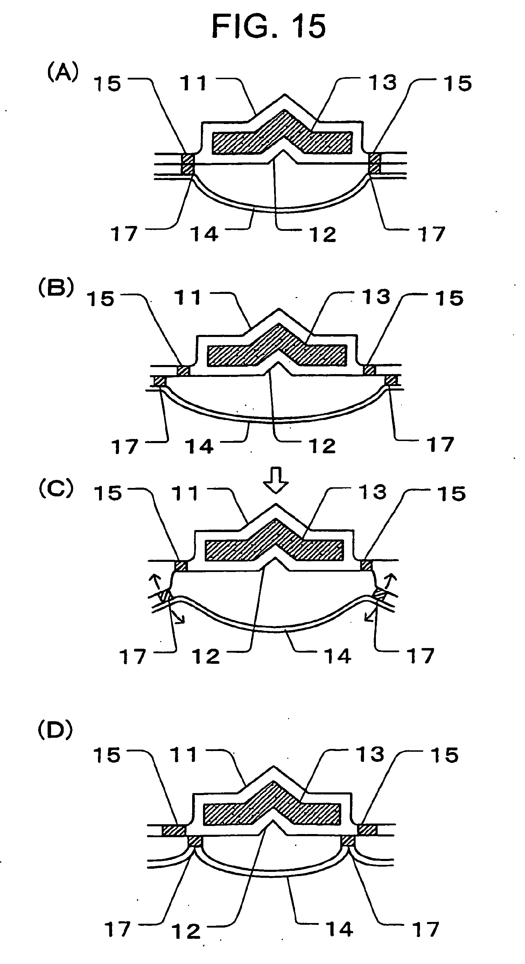

The bonding state of the restriction mini sheet piece in the lateral direction of the back side sheet will now be explained. As shown in Fig. 15(A), if the bonded section 17 is positioned at the marginal area 15 that is the bonded point between the top sheet 11 and the back side sheet 12, the marginal area 15 is hardened so as to deteriorate the wear feeling. This incident can be evaded by locating the bonded section 17 on the position other than the marginal area 15 to fix the restriction mini sheet piece 14.

However, as shown in Fig. 15(B), if the bonded section 17 is located at the position outside of the marginal area 15, there is the possibility that the stimulus is applied to the wearer due to the friction generated by the movement of the bonded section 17 and its periphery as shown in Fig.15(C).

Thus, in this embodiment, as shown in Fig. 15(D), it is desirable to stagger the positions of marginal area 15 and the bonded section 17 and locate the bonded section 17 at the position inside of the marginal area 15.

It is possible to use pressure-sensitive hot melts, thermo-sensitive hot melts, etc. to fix the restriction mini sheet piece 14 and adopt sheet pattern, linear pattern, spiral pattern, dot pattern, etc. for the application pattern.

The restriction mini sheet piece 14 may be cut in advance to conform to the fixing position shown above, or may be cut at the same time as other sheets only by making the bonding position different from other sheets.

### [Application of adhesives]

It is possible to prevent gaps from being generated between the interlabial pad 1 and the body due to the body movement of the wearer by applying an adhesive the body-contacting side of the top sheet 11 to improve the fit and contact of the interlabial pad against the interlabial space.

Adhesives usable herein include gel adhesives, etc. composed of a water-soluble polymer, a crosslinking agent, a plasticizer, water. More specifically, examples of water soluble polymers usable herein include: gelatin, sodium polyacrylic acid, polyvinyl alcohol, carboxymethyl cellulose, etc. Examples of crosslinking agents include water-soluble metal salts such as calcium chloride, magnesium sulfate. Examples of plasticizers include glycerin, wax, paraffin, etc.

Besides them, it is possible to use so-called pressure-sensitive hot melts. Examples of the pressure sensitive hot melt include those obtained by incorporating a tackifier resin such as a rosin-based resin, terpene-based resin; and plasticizers such as a wax in a main synthetic rubber resin such as styrene-isoprene-styrene block copolymers (SIS), styrene-butadiene-styrene block copolymers (SBS), styrene-ethylene-butadiene-styrene block copolymers(SEBS), styrene-ethylene-propylene-styrene block copolymers(SEPS) as a main component. Further, as another adhesive, it is also possible to use silicon resin adhesives. Example of silicon resin adhesives include mixtures composed mainly of silicon resins and fluorocarbon resins, blended with crosslinking agents made from metal salts such as platinum, molybdenum, antimony; plasticizers such as ester waxes, glycerin, machine oils.

A pressure sensitive hot melt is preferably used if application stability is also considered. More specifically, examples of heat sensitive hot melt adhesives with higher application stability include the one where a styrene block copolymer (SEBS) of 15 to 25% by weight, a flexobolizer of 15 to 35% by weight and a tackifier of 40 to 70% by weight are melted and blended. An antioxidant, a fluorescence inhibitor of 0.1 to 1.0% by weight may be added to this kind of heat sensitive hot melt adhesives. With regard to the adhesives, it is possible to prevent adhesives from being broken and peeled off during storage by coating the area of adhesives with a sheet of tissue paper (separate paper generally available) coated with a silicon resin or a sheet of film coated with a silicon resin.

Adhesives may be provided in plan pattern, dot pattern, grid pattern, stripe pattern, etc. Application positions of adhesives are not particularly limited so far as they make pad fit the body. It is particularly desirable to apply the adhesive in stripe pattern of about 1 to 5mm width near both sides of the interlabial pad considering pubic hairs near the labia (especially before the labia).

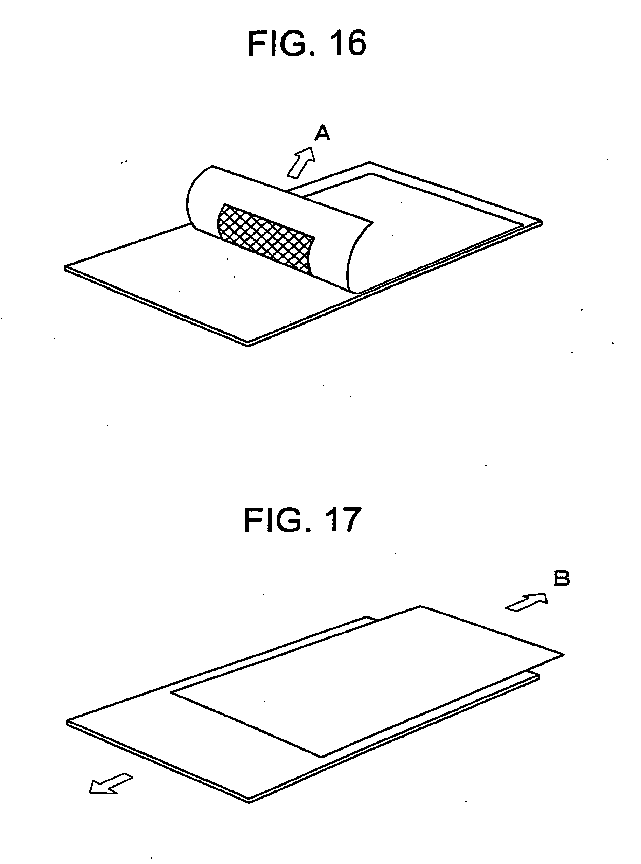

An example of evaluating methods for the adhesive force will now be explained. These evaluation methods are to measure peel strength (see Fig. 16) and shear strength (see Fig. 17) of adhesives using a constant speed stretch tensile testing machine and a stainless steel board having dimension of 80mm×50mm (length × width). Before starting an evaluation test, an adhesive was applied by 25mm × 50mm(width × length) on a polyethylene film that has almost same size as the stainless board. The film was let stand for 30 minutes under room temperature (20°C). Then, the polyethylene films was gently put on the stainless board so that the adhesive contacted the board. A roller having a weight of 2kg was rolled by just one-way. After that, a test piece was made by letting it stand under room temperature (20°C) for 30 minutes. In the peel strength test, the polyethylene film portion of the test strip was peeled off by pulling it toward the direction indicated by an arrow A in Fig. 32. In the shear strength test, the film was pulled toward the direction indicated by an arrow B in Fig.33. With regard to the test conditions, chuck interval (knob distance) was 70mm and speed of testing was 100mm/min. In this test, the measured value of the peel strength is preferably 100 to 2000mN/25mm, shear strength is preferably 2900 to 15000mN/25mm. These values are decided considering loads to the wearer's skin.

### [Other usages of interlabial pad]

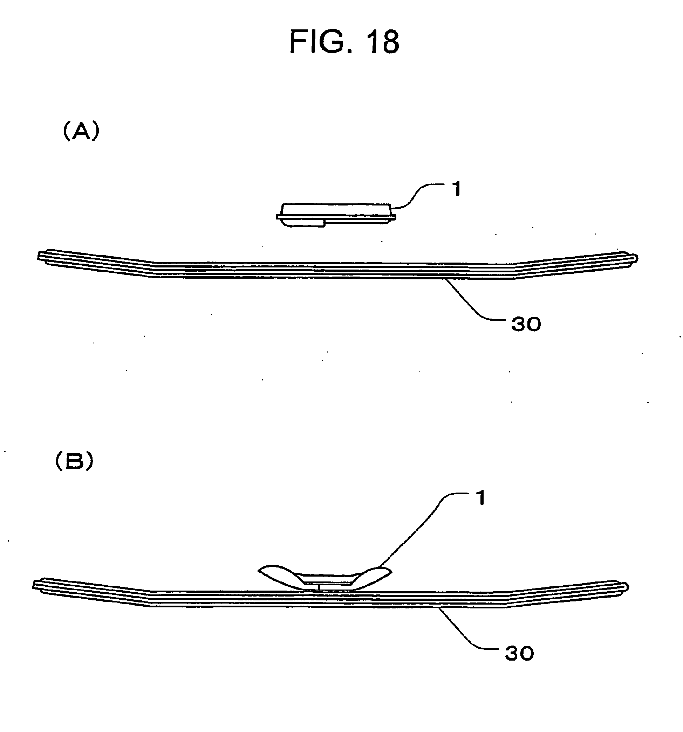

As shown in Fig.18, the interlabial pad 1 of this embodiment can be used with the ordinary sanitary napkin 30. The fixing method is that the interlabial pad 1 is fixed in the interlabial space and the sanitary napkin 30 is fixed on the undergarment. The interlabial pad 1 of this embodiment has high leakage prevention function since it is transformed according to the shape of the wearer's labia as shown in Fig.9. By using the interlabial pad with the sanitary napkin, it is possible to use the interlabial pad of the present invention more effectively even in the day when menstrual blood loss is large.

When the interlabiale pad of the present invention is wrapped individually, it is desirable to wrap the interlabial pad with a different direction against the wrapping container in such a way that directions of unwrapping and finger insertion are aligned so that the wearer can insert her finger as soon as she unwraps the wrapping container.

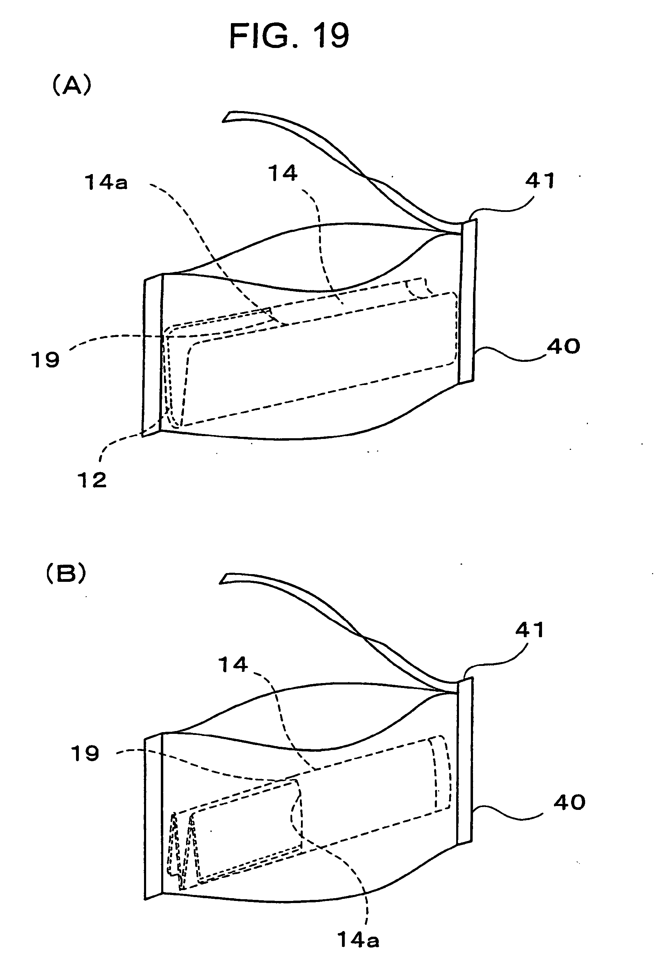

The interlabial pad 1 may be folded to be wrapped so long as the restriction mini sheet piece 14 is positioned near the unwrapping opening or finger insertion opening 19 is made to open by itself when the wrapping container is unwrapped. For example, as shown in Fig.19(A), the restriction mini sheet piece 14 may be positioned inside, or, as shown in Fig. 19(B), the restriction mini sheet piece 14 may be positioned outside.

As shown above, it is possible to further shorten the time required for wearing by individually wrapping the interlabial pad in such a way that the wearer can insert her finger into the finger insertion opening 19 formed between the restriction mini sheet piece 14 and the back side sheet 12 easily and quickly.

### [Wearing state of the interlabial pad]

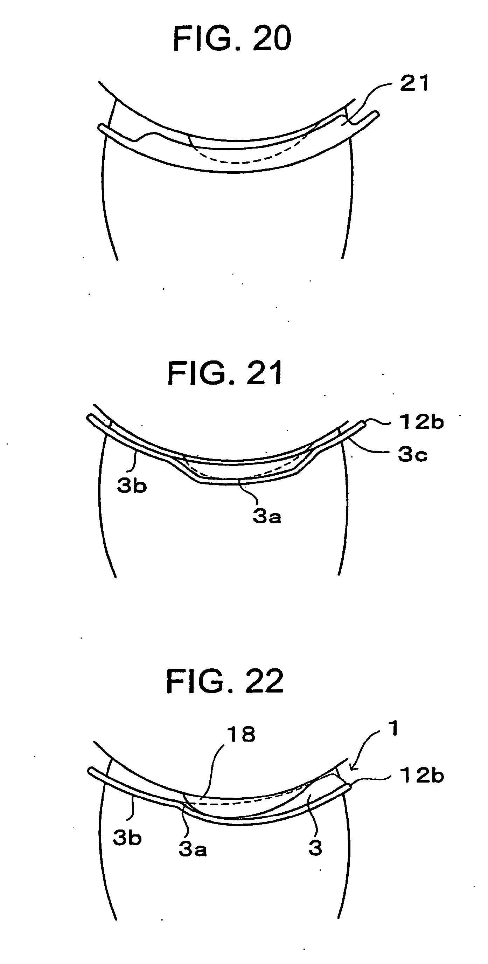

The state that the interlabial pad 1 is fixed to the wearer's labia will now be explained. Fig.20 is a schematic diagram showing the state that the interlabial pad having the ready-made projection 21 is fixed. Fig.21 is a schematic diagram showing the state that the interlabial pad having no restriction mini sheet piece at the rear end of the back side sheet is fixed (see Fig. 11). Fig.22 is a schematic diagram showing the state that the interlabial pad according to this embodiment having the restriction mini sheet piece at the rear end of the back side sheet forming a pocket shaped finger insertion space is fixed (see Fig. 12).

As shown in Fig.20, in the interlabial pad having the ready-made projection 21, the front and rear side of the projection 21 do not expand into flat shape as the interlabial pad according to this embodiment does. So, gaps will be generated between the wearer's body and the interlabial pad in the areas other than the projection 21.

On the other hand, in the interlabial pad 1 of this embodiment shown in Fig.21, in the portions corresponding to the two sections where no restriction mini sheet piece 14 is fixed, the deformed areas 3b and 3c as a part of the long convex area 3 are deformed into a flat shape at the front and rear portions of the wearer's labia. By this, there is no gap that is generated as in the case of Fig. 20.

In the interlabial pad shown in Fig.22, since the restriction mini sheet piece 14 is bonded to the rear end 12b of the back side sheet 12, only the deformed area 3b where no restriction mini sheet piece 14 exists, is deformed into a flat shape in the front area of the wearer's labia. So, this is effective for preventing leakage in users whose labia face the rear side. Thus, it is possible to broaden users' options by changing the position to fix the restriction mini sheet piece 14 accordingly.

As shown above, after the long convex area 3 has been pinched in the interlabial space, the extension area 4 is deformed and positioned to cover the pudenda as shown in Fig.23. By this, it is possible to prevent menstrual blood that has not been completely absorbed by the long convex area 3 from running off in the lateral direction of the interlabial pad.

### Industrial Applicability

As shown above, according to the present invention, since the long convex area provided on the body face side of the interlabial pad is deformed according to the shape of the wearer's labia accordingly, there is no gap between the interlabial pad and the wearer's inside of the thigh preventing menstrual blood from leaking.

In addition, with the interlabial pad of the present invention having the restriction mini sheet piece, it is possible to fix the interlabial pad in such a way that the long convex area can be easily fitted to the interlabial space resulting in A significant reduction of mis-fixing. Further, since the restriction mini sheet piece is attached to the appropriate position, the effect of preventing menstrual blood from soiling the fingertip has been improved when the pad is worn.

## Claims

1. An interlabial pad (1) with a size capable of being naturally inserted between female labia, comprising:
- a water permeable surface side sheet (11) facing a body side in use;
- a water permeable or impermeable back side sheet (12) facing a garment side in use, wherein the back side sheet (12) is bonded to the water permeable surface side sheet (11) so as to enclose an absorbent body (13) for absorbing body fluid;
- a long convex area (3) provided along a longitudinal direction of said surface side sheet (11) so that a substantial centre part of said surface side sheet (11) in a lateral direction becomes an apex towards the body side, wherein the long convex area is formed by at least an inflection portion provided by bending the surface side sheet (11) and the absorbent body (13); and
- an extended area (4) formed by an extension from a bottom of said long convex area (3) in the lateral direction of said surface side sheet (11);
**characterised in that** the interlabial pad (1) further comprises;
- a restriction mini sheet piece (14) for restricting spreading of said inflection portion, wherein the mini sheet piece (14) is bonded to the back side sheet (12) on the garment side of the back side sheet (12) in one or more bonding areas at each side in the longitudinal direction of said back side sheet (12) and is not bonded in one or more non-bonding areas in the lateral direction of said back side sheet (12), wherein at least one of said one or more non-bonding areas forms a finger insertion opening (19) between said restriction mini sheet piece (14) and said back side sheet (12); and
- a restricted part provided in the long convex area (3) where spreading of said inflection portion is restricted by the restriction mini sheet piece (14) and an unrestricted part where spreading of said inflection portion is not restricted by said restriction mini sheet piece (14).

2. The interlabial pad (1) according to claim 1, wherein the inside of said long convex area (3) forms a long hollow space (5).

3. The interlabial pad (1) according to claim 1 or 2, wherein a cross section of said long convex area (3) is substantially triangular.

4. The interlabial pad (1) according to any one of claims 1 to 3, wherein a cross sectional area of said long convex area (3) in the longitudinal direction gets gradually smaller from one end to another end of said long convex area (3).

5. The interlabial pad (1) according to any one of claims 1 to 4, wherein a cross section of said long convex area (3) is formed in a substantial triangle with a height of 5 mm to 30 mm and a bottom width of 1 mm to 20 mm.

6. The interlabial pad (1) according to any one of claims 1 to 5, wherein a length of said long convex area (3) in the longitudinal direction is 60 mm to 150 mm.

7. The interlabial pad (1) according to any one of claims 1 to 6, wherein said interlabial pad (1) is a sanitary napkin concomitant interlabial pad used together with a sanitary napkin.

8. The interlabial pad (1) according to any one of claims 1 to 7, wherein said interlabial pad (1) is an interlabiale pad (1) for incontinence.

9. The interlabial pad (1) according to any one of claims 1 to 7, wherein said interlabial pad (1) is an interlabial pad (1) for absorbing vaginal discharge.

10. A method for manufacturing the interlabial pad (1) according to any one of claims 1 to 9, wherein the interlabial pad (1) comprises an absorbent layer sheet (2) comprising said surface side sheet (11), said absorbent body (13) and said back side sheet (12) and wherein the restriction mini sheet piece (14) has a shorter width than the absorbent layer sheet (2) in the lateral direction; the method comprising the step of:
bonding the restriction mini sheet piece (14) to the absorbent layer sheet (2) at each side edge of the restriction mini sheet piece (14) and the absorbent layer sheet (2) such that a sagged portion is made on the body side of the absorbent layer sheet (2), the sagged portion forming the long convex area (3).

11. An interlabial pad (1) according to any one of claims 1 to 9 wherein said interlabial pad (1) is contained in a wrapping container for individual wrapping of the interlabial pad (1) forming a wrapping body.

12. An interlabial pad (1) according to claim 11 wherein said interlabial pad (1) is wrapped in a direction orthogonal to said wrapping container.

## Patentansprüche

1. Eine interlabiale Einlage (1), die so bemessen ist, dass sie auf natürliche Weise zwischen weibliche Schamlippen eingeführt werden kann, die Folgendes beinhaltet:
- eine wasserdurchlässige Oberflächenseitenschicht (11), die in Verwendung einer Körperseite zugewandt ist;
- eine wasserdurchlässige oder -undurchlässige Rückseitenschicht (12), die in Verwendung einer Bekleidungsseite zugewandt ist, wobei die Rückseitenschicht (12) an die wasserdurchlässige Oberflächenseitenschicht (11) gebunden ist, um einen absorbierenden Körper (13) zum Absorbieren von Körperflüssigkeit zu umschließen;
- einen langen konvexen Bereich (3), der entlang einer Längsrichtung der Oberflächenseitenschicht (11) bereitgestellt ist, so dass ein wesentlicher Mittelteil der Oberflächenseitenschicht (11) in einer lateralen Richtung zu einem Scheitel in Richtung der Körperseite wird, wobei der lange konvexe Bereich durch mindestens einen Beugungsabschnitt gebildet wird, der durch Biegen der Oberflächenseitenschicht (11) und des absorbierenden Körpers (13) bereitgestellt wird; und
- einen ausgestreckten Bereich (4), der durch eine Erstreckung von einem unteren Teil des langen konvexen Bereichs (3) in die laterale Richtung der Oberflächenseitenschicht (11) gebildet wird;
**dadurch gekennzeichnet, dass** die interlabiale Einlage (1) ferner Folgendes beinhaltet:
- ein Einschränkungsminischichtstück (14) zum Einschränken des Ausbreitens des Beugungsabschnitts, wobei das Minischichtstück (14) auf jeder Seite in der Längsrichtung der Rückseitenschicht (12) in einem oder mehreren bindenden Bereichen an der Bekleidungsseite der Rückseitenschicht (12) an die Rückseitenschicht (12) gebunden ist und in einem oder mehreren nicht bindenden Bereichen in der lateralen Richtung der Rückseitenschicht (12) nicht gebunden ist, wobei mindestens einer von dem einen oder den mehreren nicht bindenden Bereichen zwischen dem Einschränkungsminischichtstück (14) und der Rückseitenschicht (12) eine Fingereinführungsöffnung (19) bildet; und
- einen eingeschränkten Teil, der in dem langen konvexen Bereich (3) bereitgestellt ist, bei dem das Ausbreiten des Beugungsabschnitts durch das Einschränkungsminischichtstück (14) eingeschränkt ist, und einen nicht eingeschränkten Teil, bei dem das Ausbreiten des Beugungsabschnitts durch das Einschränkungsminischichtstück (14) nicht eingeschränkt ist.

2. Interlabiale Einlage (1) gemäß Anspruch 1, wobei das Innere des langen konvexen Bereichs (3) einen langen hohlen Raum (5) bildet.

3. Interlabiale Einlage (1) gemäß Anspruch 1 oder 2, wobei ein Querschnitt des langen konvexen Bereichs (3) im Wesentlichen dreieckig ist.

4. Interlabiale Einlage (1) gemäß einem der Ansprüche 1 bis 3, wobei ein Querschnittsbereich des langen konvexen Bereichs (3) in der Längsrichtung von einem Ende zu einem anderen Ende des langen konvexen Bereichs (3) nach und nach kleiner wird.

5. Interlabiale Einlage (1) gemäß einem der Ansprüche 1 bis 4, wobei ein Querschnitt des langen konvexen Bereichs (3) in einem wesentlichen Dreieck mit einer Höhe von 5 mm bis 30 mm und einer Breite des unteren Teils von 1 mm bis 20 mm gebildet ist.

6. Interlabiale Einlage (1) gemäß einem der Ansprüche 1 bis 5, wobei eine Länge des langen konvexen Bereichs (3) in der Längsrichtung 60 mm bis 150 mm beträgt.

7. Interlabiale Einlage (1) gemäß einem der Ansprüche 1 bis 6, wobei die interlabiale Einlage (1) eine Damenbinde ist, die gleichzeitig eine zusammen mit einer Damenbinde verwendete interlabiale Einlage ist.

8. Interlabiale Einlage (1) gemäß einem der Ansprüche 1 bis 7, wobei die interlabiale Einlage (1) eine interlabiale Einlage (1) für Inkontinenz ist.

9. Interlabiale Einlage (1) gemäß einem der Ansprüche 1 bis 7, wobei die interlabiale Einlage (1) eine interlabiale Einlage (1) zum Absorbieren von Scheidenausfluss ist.

10. Ein Verfahren zum Herstellen der interlabialen Einlage (1) gemäß einem der Ansprüche 1 bis 9, wobei die interlabiale Einlage (1) eine absorbierende Lagenschicht (2) beinhaltet, die die Oberflächenseitenschicht (11), den absorbierenden Körper (13) und die Rückseitenschicht (12) beinhaltet, und wobei das Einschränkungsminischichtstück (14) eine kürzere Breite als die absorbierende Lagenschicht (2) in der lateralen Richtung aufweist; wobei das Verfahren den folgenden Schritt beinhaltet:
Binden des Einschränkungsminischichtstücks (14) an jeder Seitenkante des Einschränkungsminischichtstücks (14) und der absorbierenden Lagenschicht (2) an die absorbierende Lagenschicht (2), so dass auf der Körperseite der absorbierenden Lagenschicht (2) ein herabhängender Abschnitt erzeugt wird, wobei der herabhängende Abschnitt den langen konvexen Bereich (3) bildet.

11. Interlabiale Einlage (1) gemäß einem der Ansprüche 1 bis 9, wobei die interlabiale Einlage (1) in einem Umhüllungsbehältnis zum individuellen Umhüllen der interlabialen Einlage (1), das einen Umhüllungskörper bildet, enthalten ist.

12. Interlabiale Einlage (1) gemäß Anspruch 11, wobei die interlabiale Einlage (1) in einer zu dem Umhüllungsbehältnis orthogonal verlaufenden Richtung umhüllt ist.

## Revendications

1. Une protection interlabiale (1) d'une taille à même d'être naturellement insérée entre les lèvres génitales de la femme, comprenant :
- un feuillet côté surface perméable à l'eau (11) faisant face à un côté corps lors de l'utilisation ;
- un feuillet côté dos imperméable ou perméable à l'eau (12) faisant face à un côté vêtement lors de l'utilisation, dans laquelle le feuillet côté dos (12) est collé au feuillet côté surface perméable à l'eau (11) de façon à renfermer un corps absorbant (13) destiné à absorber du fluide corporel ;
- une zone convexe longue (3) fournie suivant une direction longitudinale dudit feuillet côté surface (11) de sorte qu'une partie de centre substantielle dudit feuillet côté surface (11) dans une direction latérale vienne à former un sommet en direction du côté corps, dans laquelle la zone convexe longue est formée par au moins une portion d'inflexion fournie en pliant le feuillet côté surface (11) et le corps absorbant (13) ; et
- une zone étendue (4) formée par une extension à partir d'un bas de ladite zone convexe longue (3) dans la direction latérale dudit feuillet côté surface (11) ;
**caractérisée en ce que** la protection interlabiale (1) comprend en outre :
- un morceau formant mini-feuillet de restriction (14) destiné à restreindre l'étalement de ladite portion d'inflexion, dans laquelle le morceau formant mini-feuillet (14) est collé au feuillet côté dos (12) sur le côté vêtement du feuillet côté dos (12) dans une ou plusieurs zones collantes au niveau de chaque côté dans la direction longitudinale dudit feuillet côté dos (12) et n'est pas collé dans une ou plusieurs zones non collantes dans la direction latérale dudit feuillet côté dos (12), dans laquelle au moins une zone non collante parmi lesdites une ou plusieurs zones non collantes forme une ouverture d'insertion de doigt (19) entre ledit morceau formant mini-feuillet de restriction (14) et ledit feuillet côté dos (12) ; et
- une partie restreinte fournie dans la zone convexe longue (3) où l'étalement de ladite portion d'inflexion est restreint par le morceau formant mini-feuillet de restriction (14) et une partie non restreinte où l'étalement de ladite portion d'inflexion n'est pas restreint par ledit morceau formant mini-feuillet de restriction (14).

2. La protection interlabiale (1) selon la revendication 1, dans laquelle l'intérieur de ladite zone convexe longue (3) forme un espace creux long (5).

3. La protection interlabiale (1) selon la revendication 1 ou la revendication 2, dans laquelle une coupe transversale de ladite zone convexe longue (3) est substantiellement triangulaire.

4. La protection interlabiale (1) selon n'importe laquelle des revendications 1 à 3, dans laquelle une zone en coupe transversale de ladite zone convexe longue (3) dans la direction longitudinale devient progressivement de plus en plus petite d'une extrémité à une autre extrémité de ladite zone convexe longue (3).

5. La protection interlabiale (1) selon n'importe laquelle des revendications 1 à 4, dans laquelle une coupe transversale de ladite zone convexe longue (3) est formée en un triangle substantiel ayant une hauteur comprise entre 5 mm et 30 mm et une largeur du bas comprise entre 1 mm et 20 mm.

6. La protection interlabiale (1) selon n'importe laquelle des revendications 1 à 5, dans laquelle une longueur de ladite zone convexe longue (3) dans la direction longitudinale est comprise entre 60 mm et 150 mm.

7. La protection interlabiale (1) selon n'importe laquelle des revendications 1 à 6, dans laquelle ladite protection interlabiale (1) est une protection interlabiale concomitante d'une protection périodique utilisée conjointement à une serviette périodique.

8. La protection interlabiale (1) selon n'importe laquelle des revendications 1 à 7, dans laquelle ladite protection interlabiale (1) est une protection interlabiale (1) destinée à l'incontinence.

9. La protection interlabiale (1) selon n'importe laquelle des revendications 1 à 7, dans laquelle ladite protection interlabiale (1) est une protection interlabiale (1) destinée à absorber des pertes vaginales.

10. Une méthode destinée à la fabrication de la protection interlabiale (1) selon n'importe laquelle des revendications 1 à 9, dans laquelle la protection interlabiale (1) comprend un feuillet formant couche absorbante (2) comprenant ledit feuillet côté surface (11), ledit corps absorbant (13) et ledit feuillet côté dos (12) et dans laquelle le morceau formant mini-feuillet de restriction (14) présente une largeur plus courte que le feuillet formant couche absorbante (2) dans la direction latérale ; la méthode comprenant l'étape consistant à :
coller le morceau formant mini-feuillet de restriction (14) au feuillet formant couche absorbante (2) au niveau de chaque bord de côté du morceau formant mini-feuillet de restriction (14) et du morceau formant couche absorbante (2) de telle sorte qu'une portion fléchie soit réalisée sur le côté corps du feuillet formant couche absorbante (2), la portion fléchie formant la zone convexe longue (3).

11. Une protection interlabiale (1) selon n'importe laquelle des revendications 1 à 9 dans laquelle ladite protection interlabiale (1) est contenue dans un étui d'emballage destiné à un emballage individuel de la protection interlabiale (1) formant un corps d'emballage.

12. Une protection interlabiale (1) selon la revendication 11 dans laquelle ladite protection interlabiale (1) est emballée dans une direction orthogonale audit étui d'emballage.
